(19) **Europäisches Patentamt / European Patent Office / Office européen des brevets**

(11) **EP 1 779 773 A2**

(12) **EUROPEAN PATENT APPLICATION**

(43) Date of publication:
02.05.2007 Bulletin 2007/18

(51) Int Cl.:
*A61B 5/022* (2006.01)

(21) Application number: 06022643.8

(22) Date of filing: 30.10.2006

(84) Designated Contracting States:
AT BE BG CH CY CZ DE DK EE ES FI FR GB GR HU IE IS IT LI LT LU LV MC NL PL PT RO SE SI SK TR
Designated Extension States:
AL BA HR MK YU

(30) Priority: 01.11.2005 JP 2005318167

(71) Applicant: Omron Healthcare Co., Ltd.
Kyoto 615-0084 (JP)

(72) Inventors:
• **Sawanoi, Yukiya**
**Ukyo-ku**
**Kyoto-shi**
**Kyoto 615-0084 (JP)**

• **Eda, Kenji**
**Ukyo-ku**
**Kyoto-shi**
**Kyoto 615-0084 (JP)**
• **Tanaka, Takahide**
**Ukyo-ku**
**Kyoto-shi**
**Kyoto 615-0084 (JP)**
• **Kishimoto, Hiroshi**
**Ukyo-ku**
**Kyoto-shi**
**Kyoto 615-0084 (JP)**

(74) Representative: **Kilian, Helmut**
**Wilhelms, Kilian & Partner**
**Patentanwälte**
**Eduard-Schmid-Strasse 2**
**81541 München (DE)**

(54) **Electronic blood pressure monitor which calculates an evaluation quantity related to cardiovascular risks**

(57) A plurality of pieces of measurement result data measured in a prescribed period is retrieved from blood pressure data stored in a memory (S19). A CPU determines whether at least a plurality of prescribed number of pieces of blood pressure data corresponding to each of the plurality of measurement conditions are present in the retrieved plurality of pieces of blood pressure data (S20). When the CPU determines that at least the plurality of prescribed number of pieces of blood pressure data are present, it calculates an average value of a blood pressure data group that includes the at least the plurality of prescribed number of pieces of blood pressure data, for each of the measurement conditions (S22). The CPU then calculates a correlated evaluation quantity based on correlation of the average values respectively calculated for the measurement conditions (S24).

FIG.4

**Description**

BACKGROUND OF THE INVENTION

Field of the Invention

The present invention relates to an electronic blood pressure monitor, and in particular, to an electronic blood pressure monitor with which an evaluation quantity related to cardiovascular risks can be calculated.

Description of the Background Art

The blood pressure is one of indices for analyzing the circulatory diseases. Performing risk analysis based on the blood pressure is effective for preventing cardiovascular diseases such as cerebral apoplexy, cardiac failure, and myocardial infarction. Especially the morning hypertension, an increased blood pressure in the early morning, has relation with cardiac diseases and cerebral apoplexy. Among the morning hypertension, the symptom that the blood pressure rapidly increases during the time after one to one and half hours from getting up, called "morning surge", has been found to have a cause-effect relationship with the cerebral apoplexy. This is disclosed in detail in Kazuomi Kario, "Risk of Morning Hypertension and Cerebrovascular Disease", Journal of Blood Pressure, November issue, Sentan Igaku-sha, Nov. 1, 2002, vol. 9, no. 11, pp. 94-97. Accordingly, it is useful to recognize the correlation between the time (living habit) and the change in blood pressure for risk analysis of the cardiovascular diseases.

It is known that the blood pressure greatly varies depending on numerous physiological and environmental factors. In order to eliminate such variation, it is recommended to measure the blood pressure for a plurality of times and to determine using the average value of the measurement values. Detailed description thereof is provided in "Guidelines for the Management of Hypertension", the Japanese Society of Hypertension, December 20, 2004, pp. 7-8, and "Number of Measurements", the American Heart Association, "Hypertension", January 2005, vol. 45, pp.151-152.

Conventionally, various attempts have been made to manage the trend of blood pressure. For example, Japanese Patent Laying-Open No. 04-221528 (Patent No. 3033849) proposes a blood pressure monitor, with which blood pressure values, identification data, and the measurement date are recorded, and the blood pressure values provided with the identical identification data are selectively displayed. Also, the trend graph corresponding to the measurement situation is displayed.

On the other hand, according to Japanese Patent Laying-Open No. 04-221528, the blood pressure values are selected in accordance with the measurement time or the measurement situation that is input at the time of measurement or record. Therefore, calculation of a highly reliable evaluation quantity is not ensured, even if such technique is used.

SUMMARY OF THE INVENTION

The present invention has been made to solve the above-described problem, and an object thereof is to provide an electronic blood pressure monitor with which a highly reliable evaluation quantity can be calculated.

In order to achieve the above-described object, an electronic blood pressure monitor according to one aspect of the present invention includes: a measuring unit measuring a blood pressure of a subject; a data discriminating unit discriminating whether measured blood pressure data is calculation data for calculating an evaluation quantity; a storing unit storing the measured blood pressure data in association with at least one of a plurality of measurement conditions; a first retrieving unit retrieving a plurality of pieces of blood pressure data measured in a prescribed period from the blood pressure data stored in the storing unit; and a first determining unit determining whether at least a plurality of prescribed number of pieces of blood pressure data corresponding to each of the plurality of measurement conditions are present in the plurality of pieces of blood pressure data retrieved by the first retrieving unit. The first determining unit performs the determination for the blood pressure data discriminated as the calculation data by the data discriminating unit. The electronic blood pressure monitor further includes: a first average value calculating unit calculating, when the first determining unit determines that at least the plurality of prescribed number of pieces of blood pressure data are present, a first average value of a blood pressure data group that includes the at least the plurality of prescribed number of pieces of blood pressure data, for each of the measurement conditions; a first evaluation quantity calculating unit calculating a first correlated evaluation quantity based on correlation of the first average values respectively calculated for the measurement conditions; and a display unit displaying a calculation result of the first evaluation quantity calculating unit.

A "measurement condition" is information indicative of a physical state of the subject at the time of blood pressure measurement, and in particular, information indicative of a specific state that can be used for calculating the evaluation quantity.

Preferably, the plurality of measurement conditions include an after-waking-up time slot and a before-sleeping time slot. The blood pressure monitor further includes: a time measuring unit measuring a time point; a time point specifying unit specifying a sleep time point based on an instruction from the subject; and a condition determining unit determining to which of the plurality of measurement conditions a measurement condition at the blood pressure measurement applies, based on time point data output from the time measuring unit. The data discriminating unit includes a time determining unit determining whether a prescribed time has elapsed from the sleep time point specified by the time point specifying unit, based on the time point data output from the time measuring unit, when the condition determining unit determines that the measurement condition is the after-waking-up time slot. The data discriminating unit discriminates the measured blood pressure data as the calculation data, when the time determining unit determines that the prescribed time has elapsed.

Alternatively, it is desirable that the plurality of measurement conditions include an after-waking-up time slot and a before-sleeping time slot, and the blood pressure monitor further includes: a time measuring unit measuring a time point; and a condition determining unit determining to which of the plurality of measurement conditions a measurement condition at the blood pressure measurement applies, based on time point data output from the time measuring unit. The data discriminating unit includes a time determining unit determining whether a prescribed time has elapsed from a time point as measured in the before-sleeping time slot in the prescribed period, based on the time point data output from the time measuring unit, when the condition determining unit determines that the measurement condition is the after-waking-up time slot. The data discriminating unit discriminates the measured blood pressure data as the calculation data, when the time determining unit determines that the prescribed time has elapsed.

Preferably, the blood pressure data discriminated as the calculation data by the data discriminating unit is stored in the storing unit in association with the measurement conditions.

Preferably, based on a discrimination result of the data discriminating unit, identification information indicating whether the blood pressure data is the calculation data and the measured blood pressure data are stored in the storing unit in association with the measurement conditions.

Preferably, the first determining unit determines whether at least the plurality of prescribed number of pieces of blood pressure data, which correspond to each of the plurality of measurement conditions and in which the identification information indicates that the blood pressure data is the calculation data, are present, in the retrieved plurality of pieces of blood pressure data.

Preferably, the prescribed period is a period that is "an evaluation day" including one after-waking-up time slot and one before-sleeping time slot.

Preferably, the blood pressure monitor further includes: a second retrieving unit retrieving a plurality of blood pressure data measured in a specific period that is longer than the prescribed period, from the blood pressure data stored in the storing unit; a second determining unit determining whether at least the plurality of prescribed number of pieces of blood pressure data corresponding to each of the plurality of measurement conditions for each the prescribed period are present in the plurality of pieces of blood pressure data retrieved by the second retrieving unit; a second average value calculating unit excluding blood pressure data measured in a period determined by the second determining unit that at least the plurality of prescribed number of pieces of blood pressure data are not present, to calculate a second average value for each of a plurality of blood pressure data groups, for each of the measurement conditions; and a second evaluation quantity calculating unit calculating a second correlated evaluation quantity based on correlation of the second average values for each of the measurement conditions.

Preferably, the specific period is one of a week, a month, a season, and a year.

In order to achieve the above-described object, a blood pressure monitor according to another aspect of the present invention includes: a measuring unit measuring a blood pressure of a subject; a data discriminating unit discriminating whether measured blood pressure data is calculation data for calculating an evaluation quantity; a storing unit storing the measured blood pressure data in association with at least one measurement condition; a first retrieving unit retrieving a plurality of pieces of blood pressure data measured in a prescribed period from the blood pressure data stored in the storing unit; and a first determining unit determining whether at least a plurality of prescribed number of pieces of blood pressure data corresponding to a prescribed measurement condition are present in the plurality of pieces of blood pressure data retrieved by the first retrieving unit. The first determining unit performs the determination for the blood pressure data discriminated as the calculation data by the data discriminating unit. The electronic blood pressure monitor further includes: a first average value calculating unit calculating, when the first determining unit determines that at least the plurality of prescribed number of pieces of blood pressure data are present, a first average value of a blood pressure data group including the at least the plurality of prescribed number of pieces of blood pressure data, for the prescribed measurement condition; a first evaluation quantity calculating unit calculating a first evaluation quantity based on the first average value; and a display unit displaying a calculation result of the first evaluation quantity calculating unit.

Preferably, the prescribed measurement condition corresponds to an after-waking-up time slot. The blood pressure monitor further includes: a time measuring unit measuring time point; a time point specifying unit specifying a sleep time point based on an instruction from the subject; and a condition determining unit determining whether a measurement condition at the blood pressure measurement applies to the at least one measurement condition, based on time point data output from the time measuring unit. The data discriminating unit includes a time determining unit determining whether a prescribed time has elapsed from the sleep time point specified by the time point specifying unit, based on the time point data output from the time measuring unit, when the condition determining unit determines that the measurement condition is the prescribed measurement condition. The data discriminating unit discriminates the measured blood pressure data as the calculation data, when the time determining unit determines that the prescribed time has elapsed.

Alternatively, it is desirable that the prescribed measurement condition corresponds to an after-waking-up time slot. The blood pressure monitor further includes: a time measuring unit measuring a time point; and a condition determining unit determining whether a measurement condition at the blood pressure measurement applies to the at least one measurement condition, based on time point data output from the time measuring unit. The data discriminating unit includes a time determining unit determining whether a prescribed time has elapsed from a time point as measured in a before-sleeping time slot in the prescribed period, based on the time point data output from the time measuring unit, when the condition determining unit determines that the measurement condition is the prescribed measurement condition. The data discriminating unit discriminates the measured blood pressure data as the calculation data, when the time determining unit determines that the prescribed time has elapsed.

Preferably, the blood pressure data discriminated as the calculation data by the data discriminating unit is stored in the storing unit in association with the prescribed measurement condition.

Preferably, based on a discrimination result of the data discriminating unit, identification information indicating whether the blood pressure data is the calculation data and the measured blood pressure data are stored in the storing unit in association with the prescribed measurement condition.

Preferably, the first determining unit determines whether at least the plurality of prescribed number of pieces of blood pressure data, which correspond to the prescribed measurement condition and in which the identification information indicates that the blood pressure data is the calculation data, are present, in the retrieved plurality of pieces of blood pressure data.

Preferably, the prescribed period is a period that is an evaluation day including one the after-waking-up time slot.

Preferably, the blood pressure monitor further includes: a second retrieving unit retrieving a plurality of blood pressure data measured in a specific period that is longer than the prescribed period, from the blood pressure data stored in the storing unit; a second determining unit determining whether at least the plurality of prescribed number of pieces of blood pressure data corresponding to the prescribed measurement condition for each the prescribed period are present in the plurality of pieces of blood pressure data retrieved by the second retrieving unit; a second average value calculating unit excluding blood pressure data measured in a period determined by the second determining unit that at least the plurality of prescribed number of pieces of blood pressure data are not present, to calculate a second average value for each of a plurality of blood pressure data groups, for the prescribed measurement condition; and a second evaluation quantity calculating unit calculating a second correlated evaluation quantity based on correlation of the second average values for the prescribed measurement condition.

According to the present invention, for each measurement condition, an average value of at least a plurality of prescribed number of pieces of blood pressure data is calculated. Based on the calculated average value, the evaluation quantity is calculated. Thus, variation factors of blood pressure data dependent on psychological tension and the like of the subject at the measurement can be absorbed. Accordingly, a highly reliable evaluation quantity can be calculated.

The foregoing and other objects, features, aspects and advantages of the present invention will become more apparent from the following detailed description of the present invention when taken in conjunction with the accompanying drawings.

BRIEF DESCRIPTION OF THE DRAWINGS

Fig. 1 is an overview of an electronic blood pressure monitor according to first and second embodiments and first and second modifications of the second embodiment of the present invention.

Fig. 2 shows the internal configuration of the blood pressure monitor according to the first and second embodiments and the first and second modifications of the second embodiment of the present invention.

Figs. 3A and 3B show exemplary storage contents of measurement results in a memory according to the first and second embodiments and the second modification of the second embodiment of the present invention.

Fig. 4 is a flowchart of a main routine executed by a CPU of the electronic blood pressure monitor of the first embodiment of the present invention.

Fig. 5 is a flowchart of a subroutine showing the measurement result storing process according to the first embodiment of the present invention.

Figs. 6A-6C show exemplary screen display when the subject inputs measurement time information.

Figs. 7A and 7B show exemplary contents of a time association table.

Fig. 8 shows a first exemplary display of risk value calculation results.

Fig. 9 shows a second exemplary display of risk value calculation results.

Fig. 10 shows a third exemplary display of risk value calculation results.

Fig. 11 is a flowchart showing an interrupt process started when a risk switch is manipulated in the first embodiment.

Fig. 12 is a flowchart of a main routine executed by the CPU of the electronic blood pressure monitor of the second embodiment.

Fig. 13 is a flowchart of a subroutine showing a measurement result storing process in the second embodiment of the present invention.

Figs. 14A and 14B show exemplary configuration of a memory in the first modification of the second embodiment of the present invention.

Fig. 15 is a flowchart showing a measurement result storing process in the first modification of the second embodiment of the present invention.

Fig. 16 is a flowchart showing the flow of an interrupt process started when a sleep time switch is manipulated in the second modification of the second embodiment of the present invention.

Fig. 17 is a flowchart showing a measurement result storing process in the second modification of the second embodiment of the present invention.

DESCRIPTION OF THE PREFERRED EMBODIMENTS

Hereinafter, embodiments of the present invention will be described in detail with reference to the drawings. The same units and components have the same reference characters allotted, and detailed description thereof will not be repeated.

First Embodiment

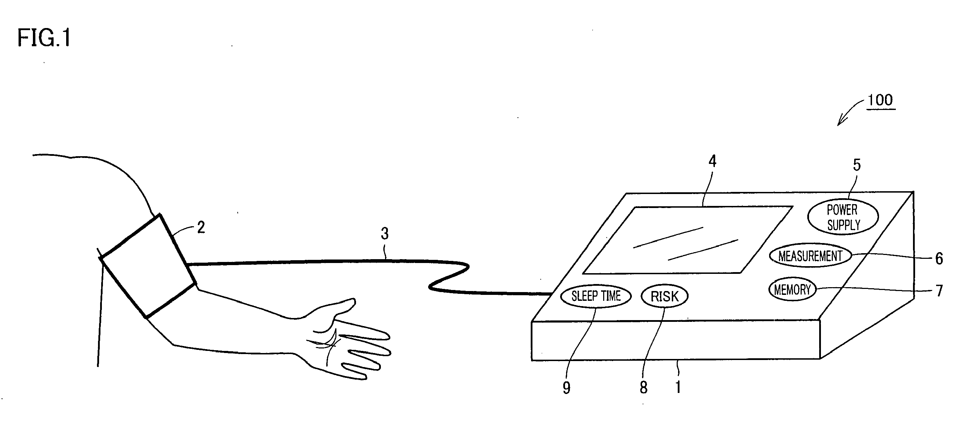

Fig. 1 is an overview of an electronic blood pressure monitor 100 according to first and second embodiments of the present invention. Referring to Fig. 1, electronic blood pressure monitor 100 of the present embodiment includes a blood pressure monitor main body 1, a cuff 2 fitted to a blood pressure measurement site of a subject and pressurized by an air pressure, and an air tube 3 connecting blood pressure monitor main body 1 with cuff 2.

Blood pressure monitor main body 1 includes a display unit 4 provided for allowing the subject to check a displayed content, and a power supply switch 5, a measurement switch 6, a memory switch 7, a risk switch 8, and a sleep time switch 9, provided for allowing the subject to externally manipulate. It is noted that sleep time switch 9 may not be provided to blood pressure monitor main body 1 of the first embodiment. Sleep time switch 9 will be described in the second embodiment later.

Power supply switch 5 is manipulated for turning ON/OFF the power supply of blood pressure monitor main body 1. Measurement switch 6 is manipulated for instructing start of blood pressure measurement. Memory switch 7 is manipulated for calling stored measurement blood pressure data (as used herein, "calling blood pressure data" means reading blood pressure data from a memory, described later, and displaying the read blood pressure data). Risk switch 8 is manipulated for instructing calculation of a risk value based on the stored blood pressure data.

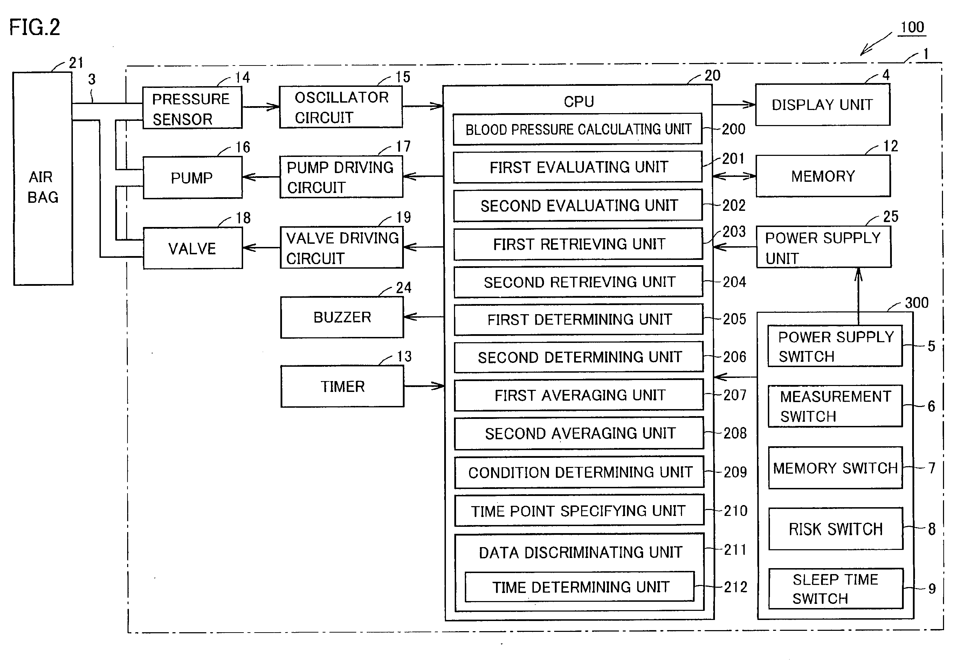

Fig. 2 shows the internal configuration of blood pressure monitor main body 1. Referring to Fig. 2, blood pressure monitor main body 1 includes a pressure sensor 14 the volume of which changes in accordance with the pressure of an air bag 21 contained in cuff 2 (hereinafter referred to as "the cuff pressure"), an oscillator circuit 15 that outputs a signal having the oscillation frequency according to the volume value of pressure sensor 14, a pump 16 and a valve 18 for adjusting the level of cuff pressure, a pump driving circuit 17 driving pump 16, and a valve driving circuit 19 for adjusting opening/closing degree of valve 18. Blood press monitor main body further includes a CPU (Central Processing Unit) 20 for centrally controlling and monitoring respective units, a display unit 4, a memory 12 storing various data and programs, a manipulation unit 300, a timer 13 that performs time-measuring operation and outputs the time data, a buzzer 24 and a power supply unit 25 for supplying power. Air bag 21 is connected to pressure sensor 14, pump 16 and valve 18 via air tube 3. CPU 20 converts a signal obtained from oscillator circuit 15 to sense the pressure.

CPU 20 performs functions of a blood pressure calculating unit 200, first and second evaluating units 201 and 202, first and second retrieving units 203 and 204, first and second determining units 205 and 206, first and second averaging units 207 and 208, a condition determining unit 209, a time point specifying unit 210, and data discriminating unit 211 having a time determining unit 212. The functions of these units of CPU 20 are implemented when CPU 20 reads and executes corresponding programs that are stored in advance in memory 12.

Manipulation unit 300 includes power supply switch 5, measurement switch 6, memory switch 7, risk switch 8, and sleep time switch 9, shown in Fig. 1.

In the above-described configuration, at the time of blood pressure measurement, blood pressure calculating unit 200 of CPU 20 applies a prescribed algorithm to the pressure data sensed based on a signal from oscillator circuit 15 to calculate blood pressure values, that is, a systolic blood pressure, a diastolic blood pressure, and a pulse rate. Conventionally available well-known procedures can be used for such measurement, and thus, detailed description thereof is not provided here. Hereinafter, thus calculated blood pressure values, or the blood pressure values and the pulse rate may also be referred to as "the measurement results". The measurement results are stored in memory 12 in association with the measurement conditions at the time of blood pressure measurement.

In the present embodiment, the measurement conditions are, as described above, the information indicative of a physical state of the subject at the time of blood pressure measurement, and in particular, the information indicative of a specific state that can be used for calculating the evaluation quantity. That is, the measurement conditions may refer to, for example, after waking up, before sleeping, after exercise, before exercise, before meal, after meal and the like. The measurement conditions may correspond to periods (for example, time slots) or to timings (for example, time, date, day of the week, month, year, season and the like).

Here, first, calculation of the evaluation quantity will be described.

As to Calculation of Evaluation Quantity

In the first embodiment and the second embodiment, which will be described later, as the evaluation quantity, for example a cardiovascular risk value is calculated. The cardiovascular risk value is considered to be useful in preventing cardiovascular events such as cerebral apoplexy, cardiac failure, cerebral infarction, cerebral hemorrhage, subarachnoid hemorrhage, transient cerebral ischemic attack, fall, syncope, vertigo, dizziness, myocardial infarction, angina pectoris, asymptomatic cardiac ischemia, arrhythmia, sudden death, dissecting aortic aneurysm, and rupture of aortic aneurysm.

As above, it is known that the blood pressure greatly varies by numerous physiological and environmental factors. Specifically, there are the variation due to progress of aging or disease, the variation dependent on time called seasonal variation and circadian variation, the variation dependent on psychological tension or physical activities, the variation synchronized with respiration, and the like. As cardiovascular risk evaluation, among such variations, it is necessary to evaluate (measure) the long-term variations of the variation due to progress of aging or disease and the seasonal variation, and the short-term variation of the circadian variation. On the other hand, if the variation dependent on psychological tension or physical activities or the variation synchronized with respiration (hereinafter, they are referred to as "psychological variations") is included in the evaluation (measurement values), the evaluation becomes less reliable and accurate risk determination becomes difficult. With a conventional electronic blood pressure monitor, such psychological variations have not been taken into account, and whether data sufficient for eliminating the psychological variations is obtained has been unclear.

Accordingly, first evaluating unit 201 of electronic blood pressure monitor 100 of the first embodiment of the present invention calculates an evaluation quantity by the following calculation method.

First Calculation Example

In the first calculation example, an evaluation quantity is calculated based on the correlation between a first blood pressure data group including at least a plurality of prescribed number of pieces of blood pressure data corresponding to an identical measurement condition among blood pressure data measured in a prescribed period and stored in memory 12, and a second blood pressure data group including at least a plurality of prescribed number of pieces of blood pressure data corresponding to another measurement condition. For example, first, an intra-group average value of blood pressure data included in the first blood pressure data group and an intra-group average value of blood pressure data included in the second blood pressure data group are calculated by first averaging unit 207. Then, for example an average value and difference value of the calculated intra-group average values are calculated as the evaluation quantities (the risk values).

That is, in the first calculation example, only when at least a plurality of prescribed number of pieces of blood pressure data measured in a prescribed period are present for each of the measurement conditions, an average value of the blood pressure data for each of the measurement conditions is calculated to obtain the evaluation quantity. It is noted that it is only necessary for the blood pressure data corresponding to each of the measurement conditions to be present in a plurality of numbers, and the number may be set differently for each of the measurement conditions.

Second Calculation Example

In the second calculation example, the evaluation quantity is calculated based on a blood pressure data group corresponding to a prescribed (one) measurement condition among blood pressure data measured in a prescribed period and stored in memory 12. In this case, first, an intra-group average value of at least a plurality of prescribed number of pieces of blood pressure data included in a blood pressure data group corresponding to a prescribed measurement condition is calculated by first averaging unit 207. Then, for example based on the calculated intra-group average value and a predetermined threshold value or a predetermined calculation formula, the evaluation quantity (the risk value) is calculated.

That is, in the second calculation example, only when at least a plurality of prescribed number of pieces of blood pressure data measured in a prescribed period are present for a prescribed measurement condition, an average value of the blood pressure data for the measurement condition is calculated to obtain the evaluation quantity.

Here, "a prescribed period" refers to an evaluation day, that is, the period of the smallest unit for calculating the evaluation quantity. In the first calculation example, the evaluation day includes each of at least two measurement conditions to be the target of evaluation quantity calculation, while in the second calculation example, the evaluation day includes one of at least one measurement condition to be the target of evaluation quantity calculation.

In the following, in the first embodiment of the present invention, the description is given assuming that the evaluation quantity related to morning hypertension is calculated in accordance with the first calculation example described above.

In this case, condition determining unit 209 of CPU 20 refers to information for specifying a measurement condition, for example, time information, to determine to which of a plurality of predetermined measurement conditions the physical status of the subject at the time of blood pressure measurement applies. In the first embodiment, the plurality of measurement conditions include, for example, two measurement conditions of an after-waking-up time slot and a before-sleeping time slot. The after-waking-up time slot and the before-sleeping time slot may be determined in advance, or may be set by the subject. In the present embodiment, it is assumed that, as information for specifying measurement conditions (the after-waking-up time slot and the before-sleeping time slot) by the subject, time information (hereinafter referred to as "measurement time information") is input and set. Thus, even when the subject is a shift worker and the like, an accurate evaluation quantity can be calculated.

As above, in the present embodiment, the evaluation quantity is calculated based on the blood pressure data measured in the after-waking-up time slot and in the before-sleeping time slot, and therefore the evaluation day is a period including one after-waking-up time slot and one before-sleeping time slot. For example, it may be a period from the start of the before-sleeping time slot until the end of the after-waking-up time slot, or may be a period from the start of the before-sleeping time slot until the start of next before-sleeping time slot. In this case, the before-sleeping time slot of the previous day and the after-waking-up time slot of today constitute the evaluation day as one set. While herein description is given assuming that a period from the start of the before-sleeping time slot until the end of the after-waking-up time slot constitutes the evaluation day, the evaluation day may be constituted by a period from the start of the after-waking-up time slot until the end of the before-sleeping time slot. That is, the after-waking-up time slot of today and the before-sleeping time slot of today may constitute the evaluation day as one set. Also, the before-sleeping time slot of today and the after-waking-up time slot of the next day may constitute the evaluation day as one set. Also, for example when the before-sleeping time slot and the after-waking-up time slot are fixed time slots, the evaluation day may correspond to 24 hours.

When the evaluation quantity is calculated by the second calculation example, the evaluation day is a period including a prescribed measurement condition, e.g., the after-waking-up time slot. For example, it may be a period from the start of the after-waking-up time slot until the end thereof, or may be a period from the start of the after-waking-up time slot until the start of next after-waking-up time slot. Also, for example when the before sleeping → after-waking-up time slot is a fixed time slot, the evaluation day may correspond to 24 hours.

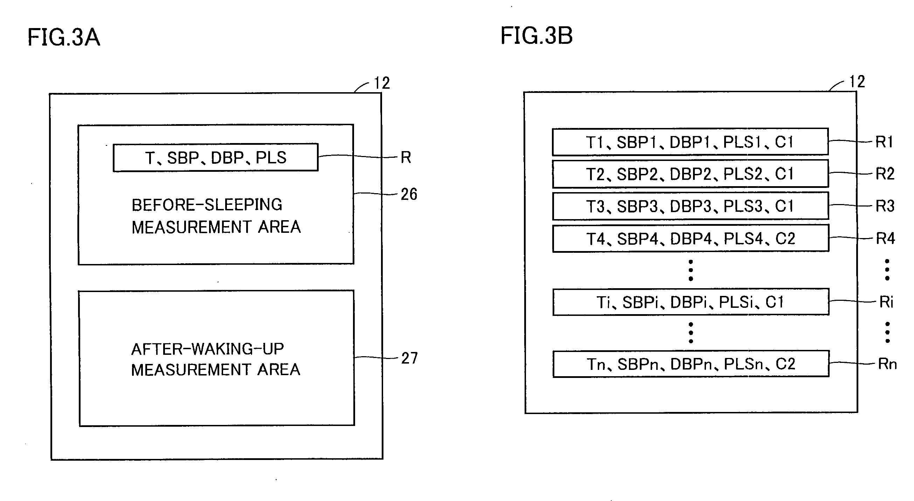

Next, a specific exemplary content of memory 12 in the first embodiment is shown in Figs. 3A and 3B.

Referring to Fig. 3A, to memory 12, division of measurement conditions, that is, storage areas 26 and 27 for the before-sleeping time slot and the after-waking-up time slot, respectively, are provided in advance. To each of areas 26 and 27, a measurement result is stored on a record R-by-record R basis. Record R includes measurement time point data T, systolic blood pressure data SBP indicative of systolic blood pressure, diastolic blood pressure data DBP indicative of diastolic blood pressure and pulse rate data PLS indicative of pulse rate.

As to measurement time point data T, data of measurement time point (the time point of starting or ending measurement) measured by timer 13 is input to CPU 20, which is then converted into measurement time point data T (year, month, day, hour, minute, day of the week) and stored in record R.

It is noted that such data is only necessary to be stored in each area as associated, and it is not limited to the storing format using record R. Also, areas 26 and 27 may be provided for each evaluation day so that measurement results may be stored in each of the applicable areas as record R. In this case, record R may not include measurement time point data T.

Referring to Fig. 3B, in memory 12, measurement results and measurement condition information are stored in pairs. In Fig. 3B, record Ri (i=1, 2, 3, ..., n) in which measurement results and measurement condition information are associated with each other is stored. In record Ri, measurement time point data Ti, systolic blood pressure data SBPi, diastolic blood pressure data DBPi, pulse rate data PSLi, and measurement condition data C1 or C2 are stored. Measurement condition data C1 and C2 correspond to measurement condition divisions, that is, the before-sleeping time slot and the after-waking-up time slot, respectively. In this example also, an area may be provided for each evaluation day so that measurement results are stored in the applicable area as record R. In this case also, record R may not include measurement time point data T.

In the following description, as shown in Fig. 3A, it is assumed that blood pressure data is grouped for each measurement condition and stored in each memory area. In the present embodiment, only the measurement results determined to be measured in the before-sleeping time slot or the after-waking-up time slot are stored in memory 12. However, a memory area may separately provided for storing measurement results measured when the state of the subject at the time of blood pressure measurement is not in the specific state (i.e., not applicable to either measurement condition), that is, measured in a time slot other than the before-sleeping time slot and the after-waking-up time slot. In this case, every time the blood pressure is measured, the measurement results are stored in any of the memory areas in memory 12. Thus, all the measured blood pressure data can be recorded.

Next, a specific evaluation quantity calculation process executed by electronic blood pressure monitor 100 of the first embodiment of the present invention is described.

CPU 20 calculates a risk value based on a program of cardiovascular risk value calculation stored in advance in an internal memory or memory 12. In order to calculate the risk value, first, first averaging unit 207 of CPU 20 performs a process of reading blood pressure data stored in memory 12 and calculating the average of a plurality of prescribed number of pieces of (for example, three) blood pressure data for each of areas 26 and 27 as shown in Fig. 3 A. That is, it calculates, for each blood pressure group, an intra-group average of a blood pressure group including a plurality of pieces of blood pressure data measured under the identical measurement condition. Then, first evaluating unit 201 calculates a risk value using the calculated average blood pressure values of intra-blood pressure data group.

The average blood pressure values are calculated using the following equations:

average of SBP measured after waking up = (data of systolic blood pressure measured after waking up SBP1 + data of systolic blood pressure measured after waking up SBP2 + ... + data of systolic blood pressure measured after waking up SBPn) / n (where n = 1, 2, 3, ...);

average of DBP measured after waking up = (data of diastolic blood pressure measured after waking up DBP1 + data of diastolic blood pressure measured after waking up DBP2 + ... + data of diastolic blood pressure measured after waking up DBPn) / n (where n = 1, 2, 3, ...);

average of SBP measured before sleeping = (data of systolic blood pressure measured before sleeping SBP1 + data of systolic blood pressure measured before sleeping SBP2 + ... + data of systolic blood pressure measured before sleeping SBPn) / n (where n = 1, 2, 3, ...);

and and

average of DBP measured before sleeping = (data of diastolic blood pressure measured before sleeping DBP1 + data of diastolic blood pressure measured before sleeping DBP2 + ... + data of diastolic blood pressure measured before sleeping DBPn) / n (where n = 1, 2, 3, ...).

The average of pulse rate is calculated using the following equations:

average of pulse rate PLS measured after waking up = (data of pulse rate measured after waking up PLS1 + data of pulse rate measured after waking up PLS2 + ... + data of pulse rate measured after waking up PLSn) / n (where n = 1, 2, 3, ...);

average of pulse rate PLS measured before sleeping = (data of pulse rate measured before sleeping PLS1 + data of pulse rate measured before sleeping PLS2 + ... + data of pulse rate measured before sleeping PLSn) / n (where n = 1, 2, 3, ...).

For calculating the risk values, the average value of blood pressure values respectively measured in the before-sleeping time slot and in the after-waking-up time slot (ME average value) and the difference between them (ME difference), which are calculated by the following equations, are used.

ME difference = average of SBP measured after waking up − average of SBP measured before sleeping

ME average = (average of SBP measured after waking up + average of SBP measured before sleeping) / 2

With electronic blood pressure monitor 100 according to the present invention, blood pressure data groups respectively including a plurality of blood pressure values measured in the before-sleeping time slot and in the after-waking-up time slot (data to be included in before sleeping area 26 and after waking up area 27) are obtained, and the average values are calculated respectively for the blood pressure data groups. Thereafter, two cardiovascular disease risk values, namely, the average value (ME average value) and difference (ME difference) between the groups, are calculated and presented (displayed) as the result.

It is noted that, not only presenting ME average value and ME difference as above, but also differences between ME average value and a predetermined threshold value (for example, 135 mmHg) and between ME difference and a predetermined threshold value (for example, 20 mmHg) may further be calculated, so that the calculated respective differences can be presented (displayed) as the risk values. Also, the risk determination result may be presented (displayed) in a prescribed displaying manner based on the calculated respective difference values. Such display of the risk determination result allows the subject to easily recognize whether the calculated risk values are higher or lower than reference values. For example, when ME difference > 20 mmHg and ME average 135 mmHg, it is determined as morning hypertension. It may also be possible to determine as morning hypertension when ME difference > 20 mmHg, as shown in Japanese Patent Laying-Open No. 04-221528. The reference values for the determination are stored in the internal memory (not shown) of CPU 20 in advance.

First evaluating unit 201 of CPU 20 may calculate, for example, a difference between the calculated intra-group average values to display the magnitude relation between the two average values as a risk determination result.

When the evaluation quantity is calculated according to the second calculation example described above, the evaluation value, that is, the risk value is calculated as follows. CPU 20 calculates the risk value based on a program of cardiovascular risk value calculation stored in advance in an internal memory or memory 12, as described above. In order to calculate the risk value, first, first averaging unit 207 of CPU 20 performs a process of reading blood pressure data stored in memory 12 and calculating the average of a plurality of prescribed number of pieces of (for example, three) blood pressure data stored in any of areas as shown in Fig. 3A. That is, it calculates an intra-group average of a blood pressure group including a plurality of pieces of blood pressure data measured under the identical measurement condition for a prescribed measurement condition, e.g., the after-waking-up time slot. Then, first evaluating unit 201 calculates a risk value using the calculated average blood pressure value of intra-blood pressure data group.

According to the second calculation example, at least the calculated average of SBP measured after waking up and average of DBP measured after waking up are calculated and presented as the risk values. Preferably, difference values between the calculated average values and respective predetermined threshold values are calculated, and the calculated difference values are presented as the risk values. As to the threshold values herein, for example, the threshold value for average of SBP measured after waking up may be set to 135 mmHg, and the threshold value for average of DBP measured after waking up may be set to 85 mmHg, based on the standard of the Joint National Committee on Prevention, Detection, Evaluation and Treatment of High Blood Pressure, or based on the hypertension standard of home blood pressure of the Japanese Society of Hypertension. Further, morning hypertension can be determined when average of SBP measured after waking up > 13 5 mmHg and average of DBP measured after waking up > 85 mmHg, and the determination result may be displayed.

Operation of Electronic Blood Pressure Monitor of First Embodiment

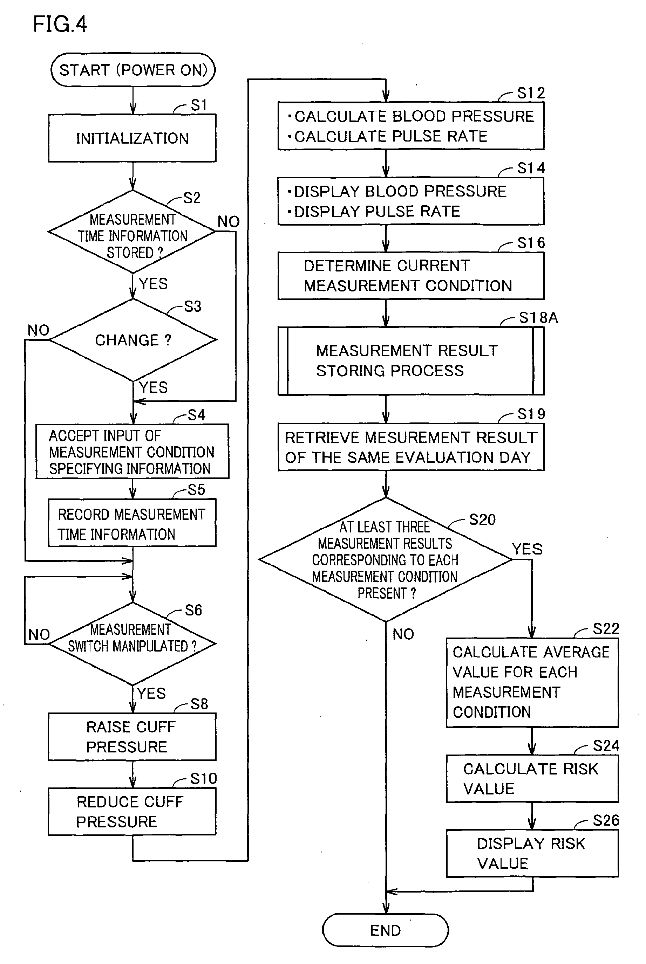

Fig. 4 is a flowchart of a main routine executed by CPU 20 of electronic blood pressure monitor 100 of the first embodiment of the present invention. The flowchart of Fig. 4 is stored in memory 12 in advance as a program, and read and executed by CPU 20. The process shown in Fig. 4 is, for example, started when power supply switch 5 is manipulated and power is supplied via power supply unit 25 to CPU 20.

Referring to Fig. 4, first, as an initializing process of electronic blood pressure monitor 100, CPU 20 controls respective units to evacuate the air in air bag 21 to thereby perform a 0 mmHg correction of pressure sensor 14 (step (hereinafter abbreviated as "S") 1). Next, CPU 20 determines whether measurement time information is stored in a time association table, described later, in memory 12 (S2). When it is stored (YES in S2), subsequently whether there is an instruction of change from the subject (S3). When the instruction of change is sensed (YES in S3), the process goes to S4. Otherwise (NO in S3), the process goes to S6.

On the other hand, if the measurement time information is not stored (NO in S2), the process goes to S4. In S3, the current measurement time information may be displayed on display unit 4 in order for the subject to determine whether change is necessary. Further, a default value may be stored in the time association table and determination process in S2 may be omitted. Thus, if the subject is of the normal life pattern, a highly reliable evaluation quantity can be calculated without inputting the measurement time information.

In S4, time specifying unit 210 of CPU 20 accepts an input of the measurement condition specifying information, that is, measurement time information. Then, CPU 20 records and updates the input measurement time information in the time association table in memory 12 (S5).

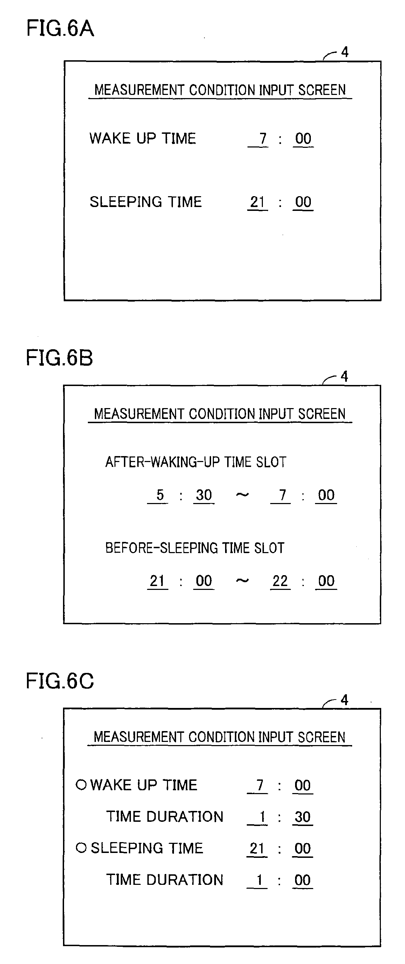

In S4, for example a screen for inputting the measurement time information is displayed on display unit 4. Specific examples of the screen being displayed are shown in Figs. 6A-6C.

Fig. 6A shows an example of accepting an input of the start or end of at least one measurement condition period (measurement time slot) as the measurement time information. On display unit 4, for example "measurement condition input screen" is displayed, and display for the subject to input wake-up time (for example, 7:00) and sleeping time (for example, 21:00) is provided. Here, it is assumed that the wake-up time is the start of the measurement condition, i.e., the after-waking-up time slot, while sleeping time is the end of the measurement condition, i.e., the before-sleeping time slot. It is assumed that both of them refer to the start, or to the end. Here, it is assumed that time duration is determined in advance separately for each of wake-up time and sleeping time, or determined in advance commonly for wake-up time and sleeping time.

Fig. 6B shows an example of accepting an input of the start and end of at least one measurement condition period as the measurement time information. On display unit 4, similarly "measurement condition input screen" is displayed, and display for the subject to input the start of wake-up time (for example, 5:30) and the end thereof (for example, 7:00) and the start of sleeping time (for example, 21:00) and the end thereof (for example, 22:00) is provided.

Fig. 6C shows an example of accepting an input of the start or end of at least one measurement condition period and a time duration (a period duration) as the measurement time information. On display unit 4, similarly "measurement condition input screen" is displayed, and display for the subject to input the wake-up time (for example, 7:00) and the sleeping time (for example, 21:00) and respective time durations (for example, 1:30 and 1:00) is provided. Herein also, it is assumed that the wake-up time is the start of the measurement condition, i.e., the after-waking-up time slot, while sleeping time is the end of the measurement condition, i.e., the before-sleeping time slot. Both may be the start, or the end.

In the present embodiment, it is assumed that one or at least two switch(es) included in manipulation unit 300 is/are used for inputting each of the times. For example, the time may be switched by one minute every time memory switch 7 is pressed, and the time being displayed when power supply switch 5 is pressed may be determined as the time where the input is made. It is noted that a not-shown switch for setting time point may be provided, with which each of the times may be input. The screen displayed when inputting the measurement time information is not limited to the manner shown in Figs. 6A-6C.

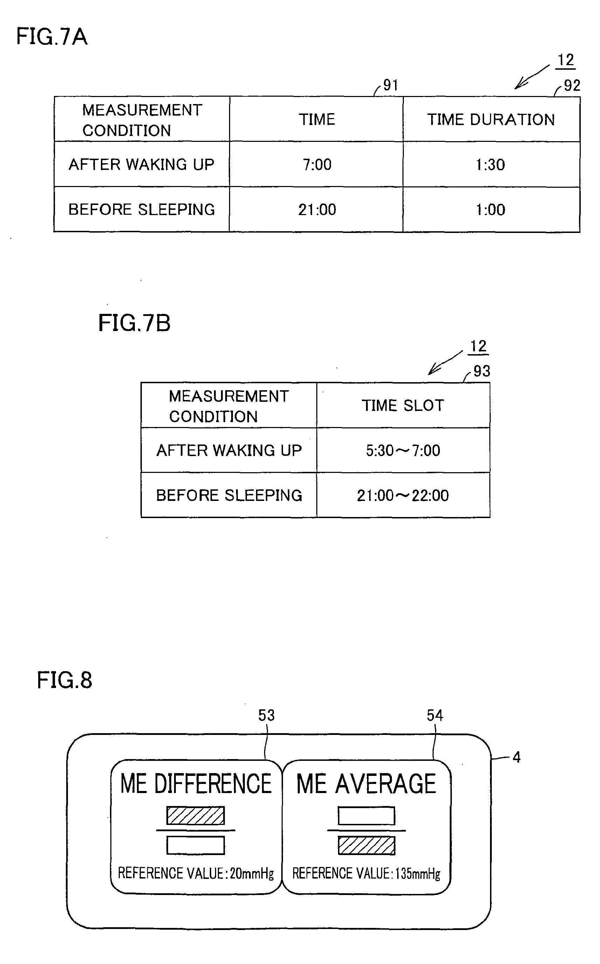

Next, exemplary contents of a time association table in which the measurement time information is stored are shown in Figs. 7A and 7B. Fig. 7A is a first exemplary content of the time association table. In the first example, in the time association table of memory 12, time data 91 and time duration data 92 are stored in association with and to form pairs with respective measurement conditions of the after-waking-up time slot and before-sleeping time slot. Time duration data 92 may be time duration data being input by the subject, or it may be predetermined time duration data.

Fig. 7B is a second exemplary content of the time association table. In the second example, in the time association table of memory 12, time slot data 93 is stored to form a pair with each of the measurement conditions of the after-waking-up time slot and before-sleeping time slot.

While in the present embodiment the measurement conditions and the measurement time information are associated with each other in the time association table, the storing manner is not limited to such a table format. Further, the time association table may be stored in a prescribed storage area other than memory 12.

Referring to Fig. 4 again, in S6, CPU 20 determines whether measurement switch 6 is manipulated. Until measurement switch 6 is manipulated, determination in S6 is repeated. When the manipulation of measurement switch 6 is sensed, the process goes to S8. It is noted that the order of performing the processes in S2-S5 and the process in S6 may be reverse.

In S8, CPU 20 controls respective units to raise the pressure to about the systolic blood pressure of the subject + 40 mmHg. Then, it gradually reduces the pressure inside air bag 21 (S10). In this pressure-reducing process, detecting the pressure inside air bag 21 with pressure sensor 14, blood pressure calculating unit 200 of CPU 20 calculates blood pressure (systolic blood pressure and diastolic blood pressure) values and a pulse rate (S12). Then, the calculated blood pressure values and pulse rate are displayed on display unit 4 (S 14). The processes for measuring blood pressure of S8-S12 are the same as those of a conventional electronic blood pressure monitor. While in the present embodiment the blood pressure measurement is performed in the pressure reducing process, it may be performed in the pressure raising process.

Next, condition determining unit 209 of CPU 20 obtains time measurement data output from timer 13 to determine the current measurement condition (S16). That is, it refers to the time association table and determines whether the current time point specified by timer 13 belongs to any of the measurement conditions, that is, divisions of measurement time slots.

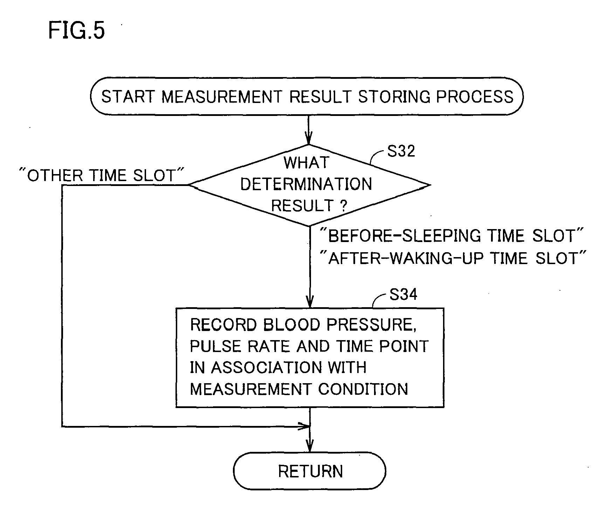

Then, CPU 20 performs a measurement result storing process (S18A). The measurement result storing process is described referring to the subroutine of Fig. 5.

Fig. 5 is a flowchart showing the measurement result storing process in the first embodiment.

Referring to Fig. 5, data determining unit 211 of CPU 20 determines what the determination result is in S16 (S32). Then, when the determination result is in the after-waking-up time slot or in the before-sleeping time slot, CPU 20 registers new record R storing the data of pressure value, pulse rate and time point, in an area of memory 12 corresponding to the measurement condition (S34).

On the other hand, when the determination result is "other time slot", that is, when it is determined that there is no applicable measurement condition, the subroutine is ended.

Referring to Fig. 4 again, first retrieving unit 203 of CPU 20 refers to each of areas 26 and 27 of memory 12 to retrieve data of measurement result of the same evaluation day (S19). Then, first determining unit 205 determines whether at least a plurality of prescribed number of pieces of, for example, three, blood pressure data corresponding to each measurement condition are present (S20). While herein the prescribed number is three, the number is not limited thereto so long as the number is at least two.

In S20, when it is determined that at least three pieces of measurement result data are not recorded for each measurement condition (NO in S20), the series of processes is ended. On the other hand, when it is determined that at least three pieces of measurement result data are recorded for each measurement condition (YES in S20), in accordance with the procedure described above, by first averaging unit 207, an average value of blood pressure data for each measurement condition is calculated, that is, respective average values of data groups of areas 26 and 27 are calculated (S22). Then, by first evaluating unit 201, ME average value and ME difference are calculated as the cardiovascular disease risk values (S24). It is noted that, in S24, a difference between ME average value and a predetermined reference value as well as a difference between ME difference and a predetermined reference value may further be calculated. Then, the risk value calculation result in S24 is displayed on display unit 4 (S26).

Thus, the series of process is ended.

While in the present embodiment the calculation of the average value and the risk value are performed as calculation of the evaluation quantity, at least the average value may be calculated. That is, the average value may be presented (displayed) to the subject as the risk value.

Here, an exemplary display of the risk value calculation results in S26 is shown.

Fig. 8 shows a first exemplary display of risk value calculation results (risk determination results). Display areas 53 and 54 of display unit 4 respectively display the calculated ME difference and ME average value as the risk determination results. Each of display areas 53 and 54 displays upper and lower two blocks, between which a line is horizontally displayed. The line indicates a reference value. Therefore, for example when each of the calculated values is higher than the reference value, the block over the line is shown in reversed display (filled display) while the block below the line is shown in non-reversed display (blank display). When it is lower than the reference value, the block below the line is shown in reversed display while the block over the line is shown in non-reversed display. In the example of Fig. 8, it is shown that the calculated ME difference is higher than the reference value (20 mmHg) while the calculated ME average is lower than the reference value (135 mmHg).

The displaying manner of the risk value calculation results is not limited to the manner of Fig. 8. For example, the calculated ME difference value and ME average value may separately be displayed.

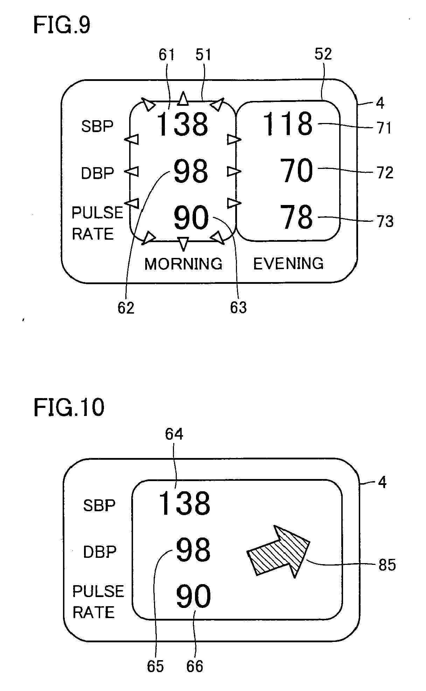

Also, a difference between respective average values of measurement conditions may be obtained in S24 and the magnitude relation between them may be displayed in S26. Fig. 9 shows a second exemplary display of the risk value calculation results, showing an exemplary display of correlation between the average values obtained from blood pressure data measured under respective different measurement conditions. In Fig. 9, display unit 4 includes display areas 51 and 52 for respectively displaying after-waking-up measurement data and before-sleeping measurement data, as disposed next to each other. On display area 51, data of average of SBP measured after waking up 61, data of average of DBP measured after waking up 62, and data of average of pulse rate PLS measured after waking up 63 obtained by the aforementioned equations are displayed. Next to them, respectively, on display area 52, data of average of SBP measured before sleeping 71, data of average of DBP measured before sleeping 72, and data of average of pulse rate PLS measured before sleeping 73 are displayed. Thus, measurement data of display area 51 and that of display area 52 are displayed in a correlated manner.

As to the display of Fig. 9, CPU 20 compares the data displayed on display area 51 with that displayed on display area 52. Based on the comparison result, CPU 20 may implement displaying in a prescribed manner, such as changing the display color for higher blood pressure data to a prescribed color being different from that for lower blood pressure data. For example, CPU 20 may compare data of average of SBP measured after waking up 61 with data of average of SBP measured before sleeping 71 and display data indicative of the higher blood pressure all in a prescribed color. Fig. 9 is an exemplary display of changing color when data of average of SBP measured after waking up 61 is higher. The displaying manner is not limited to the color changing display, and may be changed to blinking display.

As above, in the present embodiment, since the period during which the data of blood pressure of which evaluation quantity is to be calculated is divided by evaluation day basis, the reliability of the evaluation quantity can be improved. Further, the evaluation quantity is calculated only when a plurality of pieces (for example, three) of blood pressure data corresponding to each measurement condition are present, the effect of the psychological variations on the evaluation quantity can be absorbed.

Further, since the measurement condition at the pressure measurement is determined based on the time point data from timer 13 and the measurement time information input by the subject, highly accurate evaluation quantity calculation in accordance with the life cycle of the subject is implemented, irrespective of the actual time point.

Still further, once the subject sets the measurement time information, it is not necessary for the subject to input the measurement time information from the second time, and therefore the subject of a regular life cycle can be freed from the trouble of operation. From the second time, and when it is not necessary to make a change, time for a series of processes related to the blood pressure measurement can be shortened. Still further, since the time slot corresponding to each measurement condition is fixed (so long as there is no instruction of change) in the first embodiment, the highly reliable evaluation quantity can be presented particularly to the subject of a stable life cycle.

It is noted that, information for specifying a measurement condition may be input by the subject every time the measurement is performed. For example, a not-shown switch corresponding to each measurement condition may be provided so that information on the measurement condition corresponding to each switch may be accepted every time the measurement is performed.

While in the first embodiment the measurement time information is input before measuring the blood pressure (before calculating the blood pressure), it may be input after measuring the blood pressure (after calculating the blood pressure).

While in the present embodiment processes of S20-S26 are performed every time the blood pressure is measured, the main routine may be ended at the process of S18A, and the processes of S20-S26 may be performed only in an interrupt process. The processes of S20-S26 may also be performed only when the subject presses a prescribed switch, for example.

In the present embodiment, it is assumed that an interrupt process is performed when memory switch 7 is manipulated between prescribed steps. In the interrupt process, for example the measurement results stored in memory 12 are sequentially read and displayed on display unit 4. The above S20-S26 processes may be performed in the interrupt process also to display the risk values.

When risk switch 8 is provided to electronic blood pressure monitor 100, the manipulation of risk switch 8 may display a risk calculation result of a specific period longer than the evaluation day, for example on a week-by-week basis. Even when risk switch 8 is not provided, this may be performed for example when memory switch 7 is pressed and a prescribed manipulation is performed. A "specific period" is a period that is longer than the evaluation day and that includes a plurality of evaluation days.

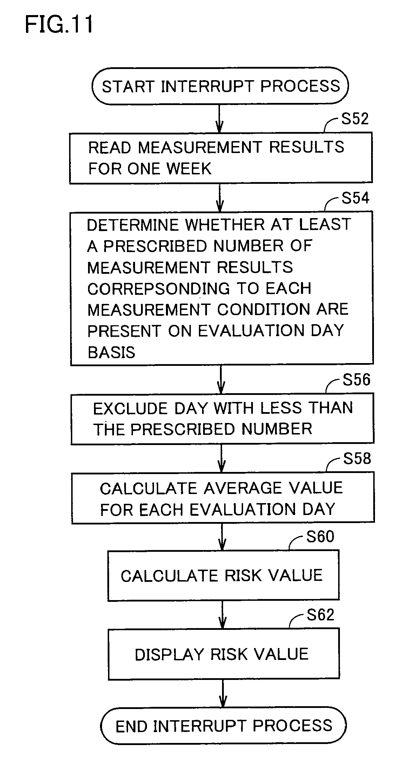

Fig. 11 is a flowchart showing an interrupt process that is started when risk switch 8 is manipulated.

Referring to Fig. 11, first determining unit 205 of CPU 20 reads measurement results for the past one week recorded in memory 12 (S52). Then, it determines whether at least three measurement results corresponding to each measurement condition are present, on evaluation day basis (S54), and excludes the evaluation day with less than three measurement results (S56).

First averaging unit 207 calculates an average value of blood pressure data for each measurement condition, only for the evaluation day with at least three pieces of blood pressure data for each measurement condition (S58). For example, it may calculate the average value for each evaluation day, and then further average the average values of the evaluation days so that the average value of one week is obtained. It may also possible to collectively calculate, for all the evaluation days other than those excluded in S56, the average of blood pressure data of the after-waking-up time slot and the average of blood pressure data of the before-sleeping time slot.

Next, first evaluating unit 201 of CPU 20 calculates the risk value on a week-by-week basis based on the average value calculated in S58 (S60), and displays the risk value (S62). The processes in S60 and S62 are the same as in S24 and S26 in Fig. 4, respectively, and therefore description thereof is not repeated.

Thus, when calculating the risk value on a week-by-week basis including a plurality of evaluation days, by increasing the accuracy for each evaluation day, the reliability of the evaluation quantity on a week-by-week basis can consequently be improved.

While in the present embodiment the risk value on a week-by-week basis is calculated and displayed when risk switch 8 is manipulated, as described above, the period is not limited to a week so long as it includes a plurality of evaluation days. For example, the risk value may be calculated on a day of the week-by-day of the week basis, a month-by-month basis, a year-by-year basis, a season-by-season basis and the like. Additionally, a plurality of risk switches 8 may be provided respectively corresponding to a week-by-week basis, a month-by-month basis, and a year-by-year basis.

When the evaluation quantity is calculated according to the second calculation example also, it can be implemented by the similar procedure as described above. In this case, in S4, only the information for specifying a prescribed measuring condition, e.g., the after-waking-up time slot, may be input. In S20, whether at least three measuring results corresponding to the prescribed measurement condition are present is determined. In S22, the average value for the prescribed measurement condition is calculated.

When the evaluation quantity is calculated according to the second calculation example, in S26, whether the average value of the blood pressure data corresponding to the prescribed measurement condition is higher or lower than, for example, a prescribed reference value, may be displayed. Determination of the value being higher or lower than the reference value is made by first evaluating unit 201 of CPU 20. Specifically, when it is determined that the relational expression "systolic blood pressure > 135 mmHg or diastolic blood pressure > 85 mmHg" or "systolic blood pressure > 135 mmHg and diastolic blood pressure > 85 mmHg" is satisfied, then it is evaluated that the blood pressure value is higher than a prescribed reference value. When it is determined that the relational expression is not satisfied, then it is evaluated that the blood pressure value is lower than a prescribed reference value. In the relational expression, one of or both of data of average of SBP measured after waking up 61 and data of average of DBP measured after waking up 62 is/are applied as to the measurement condition of the after-waking-up time slot, and one of or both of data of average of SBP measured before sleeping 71 and data of average of DBP measured before sleeping 72 is/are applied as to the measurement condition of the before-sleeping time slot.

Fig. 10 shows a display example of the risk calculation result for the blood pressure data in the after-waking-up time slot. In Fig. 10, data of average of SBP measured after waking up 64, data of average of DBP measured after waking up 65, and average of pulse rate PLS measured after waking up 66 within one evaluation day, as well as an arrow 85, are shown. CPU 20 compares data of average of SBP measured after waking up 64, data of average of DBP measured after waking up 65, and average of pulse rate PLS measured after waking up 66 with respective reference values, and indicates by arrow 85 whether morning hypertension is presumed. Fig. 10 shows the risk determination result of the case where morning hypertension is presumed, with arrow 85 pointing upwardly. When morning hypertension is not presumed, arrow 85 points downwardly.

Further, when the evaluation quantity is calculated according to the second calculation example, in the interrupt process shown in Fig. 11, in S54, whether at least three measurement results corresponding to a prescribed measurement result are present is determined on an evaluation day-by-evaluation day basis. In S58, the average value for the prescribed measurement condition is calculated.

Second Embodiment

Next, a second embodiment of the present invention is described. The electronic blood pressure monitor according to the second embodiment is similarly structured as in the first embodiment, and therefore description is given using the reference characters for electronic blood pressure monitor 100 in Figs. 1 and 2 herein also. In the second embodiment also, description is given assuming that the evaluation quantity is calculated according to the first calculation example described above.

In contrast to the first embodiment where all blood pressure data measured under the measurement condition to be the target of evaluation quantity calculation (herein after also referred to as "a specific measurement condition") is regarded as data for calculating the evaluation quantity (hereinafter referred to as "calculation data"), in the second embodiment, even the blood pressure data measured under the specific measurement condition is not employed as the calculation data, if it does not satisfy a prescribed condition. That is, only when the blood pressure is measured under a specific measurement condition and presumed to satisfy a prescribed condition, the data of the measured blood pressure is used as the calculation data. It is noted that "when it does not satisfy a prescribed condition" is the case where it is presumed that the blood pressure measurement value is affected by psychological variation, e.g., when the subject is deprived of sleep.

For example, when an input of information for specifying a measurement condition is accepted every time the blood pressure is measured, it may be applicable to a specified measurement condition irrespective of the measurement time point. However, even when it is determined that the measurement is carried out in the after-waking-up time slot, the reliability of the measurement result itself may be low if the subject is extremely deprived of sleep. In the present embodiment, when sleep deprivation is presumed, the data is not treated as calculation data and whereby a further reliable evaluation quantity can be calculated.

In the following, an operation of electronic blood pressure monitor 100 of the second embodiment is described.

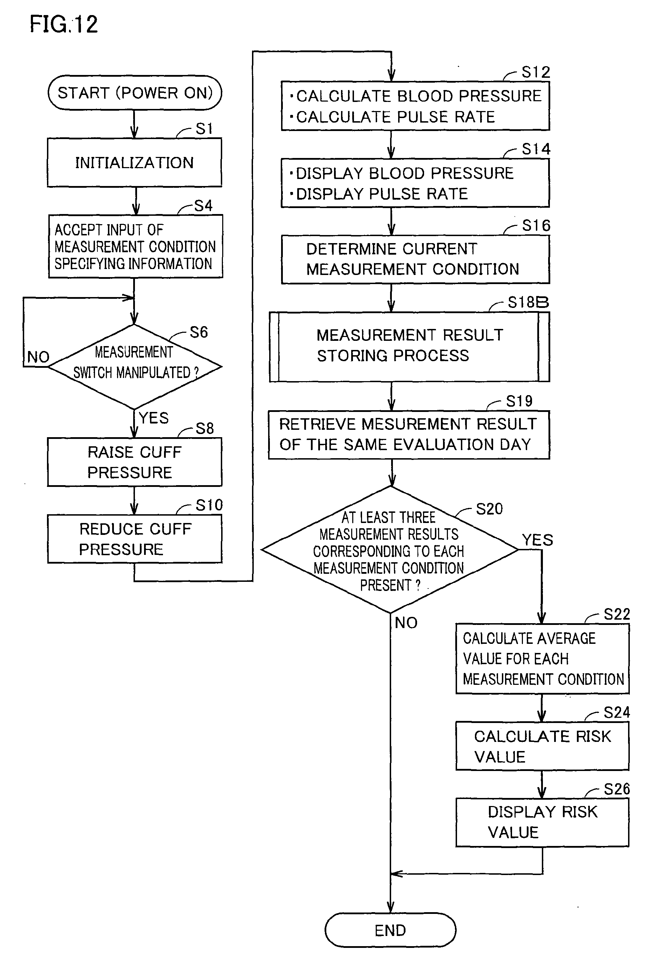

Fig. 12 is a flowchart of a main routine executed by CPU 20 of electronic blood pressure monitor 100 of the second embodiment. The flowchart of Fig. 12 is stored in memory 12 as a program in advance, and read and executed by CPU 20. The processes similar to those in the flowchart of Fig. 4 have the same reference characters allotted and description thereof is not repeated.

Referring to Fig. 12, after an initialization process in S1 is ended, CPU 20 accepts an input of measurement condition specifying information, i.e., measurement time information (S4). In the second embodiment, in S4, CPU 20 temporarily records the accepted measurement time information in the internal memory. Then, after the process of S4 is ended, the processes of S6-S 14 described above are sequentially performed. In S 14, a blood pressure value and a pulse rate are displayed on display unit 4. In S 16, based on time point data output from timer 13 and the measurement time information input in S4, a present measurement condition is determined by condition determining unit 209.

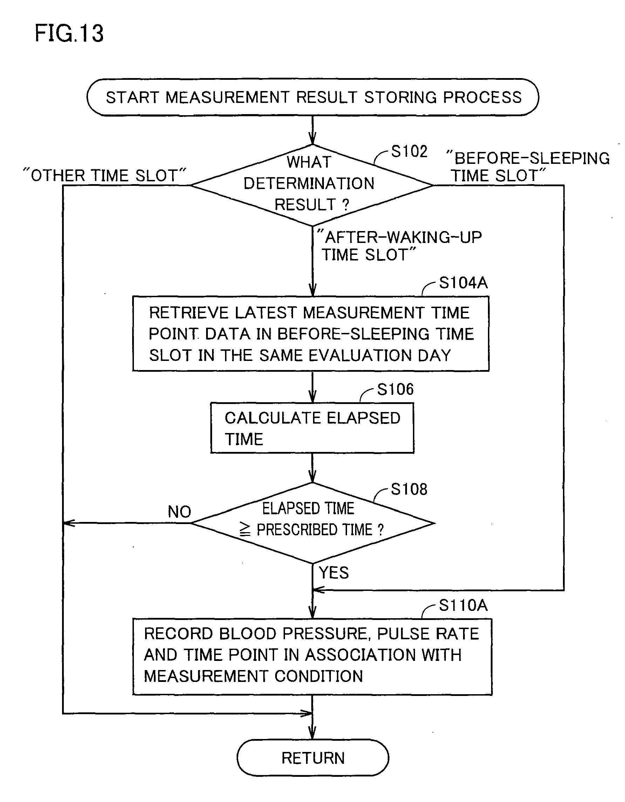

After the process in S 16 is ended, a measurement result storing process is executed (S 18B). The measurement result storing process will be described using a subroutine of Fig. 13.

Fig. 13 is a flowchart showing the measurement result storing process in the second embodiment. Referring to Fig. 13, first, condition determining unit 209 of CPU 20 determines what a determination result is in S 16 (S102). When the determination result is the before-sleeping time slot, the process goes to S110A. When the determination result is the after-waking-up time slot, the process goes to S 1 04A. On the other hand, when the determination result is "other time slot", the subroutine is ended. The processes shown in S102 and S110A correspond to those in S32 and S34 in Fig. 5, respectively.

In S104A, time point specifying unit 210 and time determining unit 212 of CPU 20 retrieve, for example, the latest (last) measurement time point data in the before-sleeping time slot in the same evaluation day stored in memory 12. Based on the time point data output from timer 13, an elapsed time is calculated (S106). That is, how many hours have passed since the last measurement time point in the before-sleeping time slot in the same evaluation day is calculated. Then, whether the elapsed time is at least a prescribed time (for example, four hours) is determined (S108). When it is at least a prescribed time (YES in S 108), the process goes to S 110A.

In S110A, CPU 20 stores in memory 12 data of pressure value, pulse rate and time point in association with the measurement condition.

On the other hand, when the elapsed time is less than the prescribed time (NO in S 108), the subroutine is ended without recording the measurement result.

Referring again to Fig. 12, when the measurement result storing process of S18B is ended, the processes of S19-S26 as described above are performed, and the series of processes is ended.

Thus, in the second embodiment, presuming the last measurement time point in the before-sleeping time slot

14

of the previous day as the sleep time point, whether the subject is deprived of sleep is determined. Then, when the subject is determined to be deprived of sleep, the measurement result is not stored in memory 12 and thus can be excluded from the target of evaluation quantity calculation. This can further eliminate variation in the blood pressure value due to sleep deprivation or the like.

In the flowchart shown in Fig. 13, while the description have been given assuming that the measurement result in the before-sleeping time slot in the same evaluation day is stored, when the measurement result in the before-sleeping time slot in the same evaluation day is not stored, for example the processes of S 106 and S 108 may be skipped and the process of S 110A may be performed. Alternatively, since the physical state of the subject at the measurement is unknown if the measurement result in the before-sleeping time slot in the same evaluation day is not stored, the routine may be ended without performing the processes of S106-S110A.

Further, when an evaluation quantity as to the after-waking-up time slot is calculated according to the second calculation example also, the process similar to that described above can be performed by providing the after-waking-up time slot that is a specific measurement condition and the before-sleeping time slot that is not a specific measurement condition, and storing blood pressure data measured in the sleeping time slot. In this case, similarly to the case where the evaluation quantity is calculated according to the first calculation example, it is assumed that the evaluation day includes one before-sleeping time slot and one after-waking-up time slot.

In the second embodiment also, the process similar to the interrupt process shown in Fig. 11 in the first embodiment may be performed. Thus, also on a specific period-by-period basis, such as on a week-by-week basis, further reliable evaluation quantity can be calculated.

The measurement result storing process shown in Fig. 13 may be performed in S 18A in Fig. 4 of the first embodiment.

While in the foregoing description the measurement result itself is not stored if it does not satisfy a prescribed condition, it may also be possible to perform the following process.

First Modification

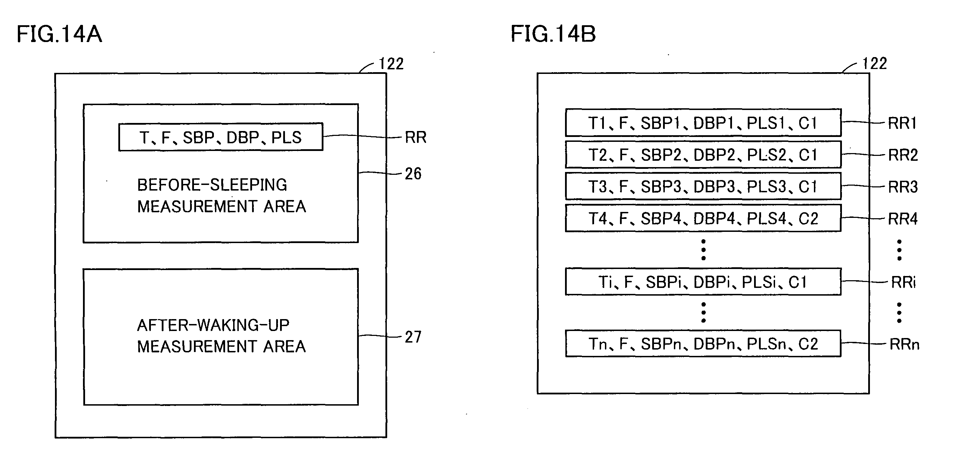

In a first modification of the second embodiment, identification information indicative of whether a prescribed condition is satisfied is recorded in association with measurement result data. In the first modification, electronic blood pressure monitor 100 of the second embodiment has a memory 122 (see Figs. 14A and 14B) in place of memory 12.

The electronic blood pressure of the first modification of the second embodiment is similarly structured as the first embodiment, except for memory 122, and therefore the reference characters for electronic blood pressure 100 in Figs. 1 and 2 are also used for description herein.

Figs. 14A and 14B show exemplary configuration of memory 122 in the first modification of the second embodiment. Referring to Fig. 14A, in memory 12, similarly to the exemplary configuration shown in Fig. 3, division of measurement conditions, that is, storage areas 26 and 27 for the before-sleeping time slot and the after-waking-up time slot, respectively, are provided in advance. To each of areas 26 and 27, a measurement result is stored on a record RR-by-record RR basis. Record RR includes measurement time point data T, systolic blood pressure data SBP, diastolic blood pressure data DBP, pulse rate data PLS, and a calculation flag F that is identification data indicative of whether it is risk calculation data.

In the first modification of the second embodiment also, it is not limited to the exemplary configuration of Fig. 14A and it may be the configuration as in Fig. 14B.

Referring to Fig. 14B, in memory 122, measurement results and measurement condition information are stored in pairs. That is, in Fig. 14B, record RRi (i=1, 2, 3, ..., n) in which measurement results and measurement condition information are associated with each other is stored for every blood pressure measurement. In record RRi, measurement time point data Ti, systolic blood pressure data SBPi, diastolic blood pressure data DBPi, pulse rate data PSLi, measurement condition data C1 or C2, and calculation flag F are stored.

In the first modification of the second embodiment also, the processes basically similar to those shown in the flowchart of Fig. 12 are performed by CPU 20. It is different from the second embodiment in the processes of S18B, S20B and S22. Accordingly, only those processes different from the second embodiment are described in the following.

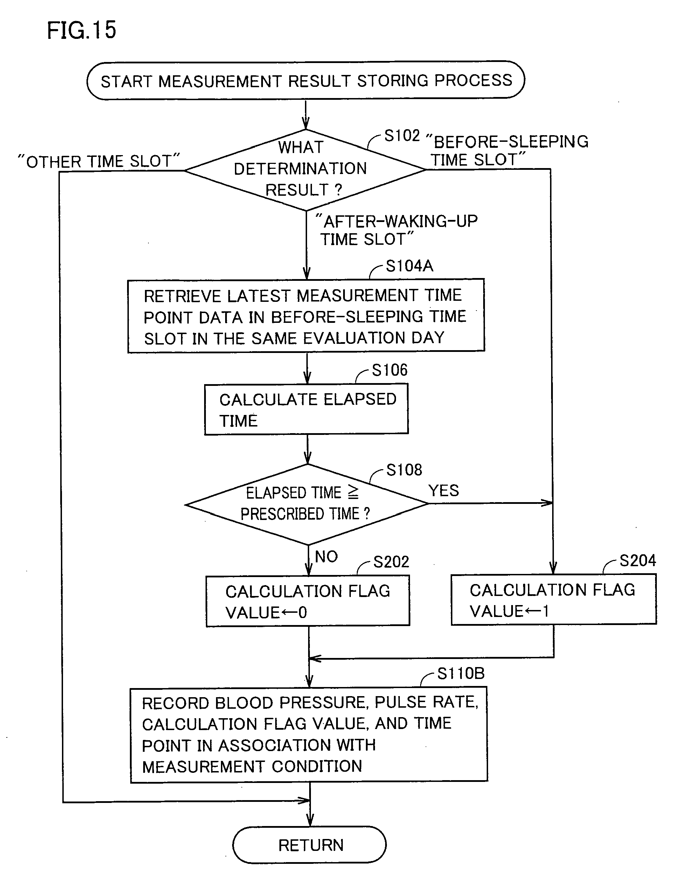

Fig. 15 is a flowchart showing a measurement result storing process in the first modification of the second embodiment. The processes similar to those shown in the flowchart of Fig. 13 have allotted the same step numbers, and detailed description thereof is not repeated.

Referring to Fig. 15, similarly to the second embodiment, first, condition determining unit 209 of CPU 20 determines what a determination result is in S 16 (S102). When the measurement condition is the before-sleeping time slot, the process goes to S204. On the other hand, when the measurement condition is "other time slot", that is, when it is determined that there is no applicable measurement condition, the subroutine is ended.

When the measurement condition is the after-waking-up time slot, the processes of S104A-S108 described above are performed. In S108, when the elapsed time is at least a prescribed time (YES in S 108), the process goes to S204. On the other hand, if the elapsed time is less than the prescribed time (NO in S108), the process goes to S202.

In S202, CPU 20 sets the value of calculation flag F to 0, and temporarily records the calculation flag F being set to 0. On the other hand, in S204, CPU 20 sets the value of calculation flag to 1, and temporarily records the calculation flag F being set to 1.

After the process of S202 or S204 is ended, CPU 20 stores in memory 122 data of pressure value, pulse rate, calculation flag value and time point in association with the measurement condition (S110B). Thus, the subroutine is ended.

As above, in the first modification, all the blood pressure data measured under one of the predetermined measurement conditions is stored. This makes it possible to use the measurement results for a purpose other than the risk value calculation. For example, the measurement results may be sequentially read and displayed.

Next, description is given on processes performed in S20 and S22 in Fig. 12 in a second modification of the second embodiment, in place of the processes described in the second embodiment.

In the second modification, in S20, second retrieving unit 204 and second determining unit 206 determines whether at least three measurement results wherein calculation flag F=1 corresponding to each measurement condition are present. In the following, such a measurement result wherein calculation flag F=1 is referred to as "a calculable measurement result". That is, whether at least three calculable measurement results are stored for each of the before-sleeping time slot and the after-waking-up time slot is determined. If one of the time slots fails to store the at least three calculable measurement results, then the series of processes is ended. On the other hand, when at least three calculable measurement results for each of the measurement conditions are stored, the process goes to S22.

In the second modification, in S22, second averaging unit 208 of CPU 20 calculates the average value for the calculable measurement results for each measurement condition. Then, based on the average values thus calculated, the processes of S24-S26 including calculation of the evaluation quantity using second evaluation unit 202 are performed.

While in the first modification the calculation flag value (0 or 1) is stored in association with the measurement result even when the measured condition is the before-sleeping time slot, the calculation flag value may not be stored.

When the evaluation quantity is calculated according to the second calculation example, in S20, whether at least three calculable measurement results corresponding to the after-waking-up time slot in the same evaluation day are stored is determined. If at least three calculable measurement results are not stored, the series of processes are ended. If at least three calculable measurement results are stored, the process goes to S22. It is noted that, when the evaluation quantity is calculated according to the second calculation example, if the measurement condition is the before-sleeping time slot, the calculation flag value may be set to 0 in S204.

In the first modification of the second embodiment also, the process similar to the interrupt process shown in Fig. 11 in the first embodiment may be performed. That is, in S54, CPU 20 determines whether at least three measurement results wherein calculation flag F=1 are present. Thus, as to the evaluation quantity of the week-by-week basis also, further reliable evaluation quantity can be calculated.

The measurement result storing process shown in Fig. 15 may be performed in S18A shown in Fig. 4 of the first embodiment.

Second Modification

In the second embodiment and its first modification, by assuming the time of the latest time point data in the before-sleeping time slot stored in the same evaluation day is assumed to be the sleep time point, whether a prescribed condition is satisfied is determined. In the second modification of the second embodiment, by accepting an input of information for specifying the sleep time point, whether a prescribed condition is satisfied is determined. The "sleep time point" herein refers to the time point at which the subject is assumed to fall asleep.

The electronic blood pressure monitor of the second modification of the second embodiment is similarly structured as that of the first embodiment, description is given using the reference characters for electronic blood pressure monitor 100 in Figs. 1 and 2 herein also.

Electronic blood pressure monitor 100 in the second modification of the second embodiment includes, for example, a sleep time switch 9. Time point specifying unit 210 of CPU 20 specifies the time point at which sleep time switch 9 is manipulated as the sleep time point. In the second modification, it is assumed that an interrupt process to the main routine shown in Fig. 4 is performed.

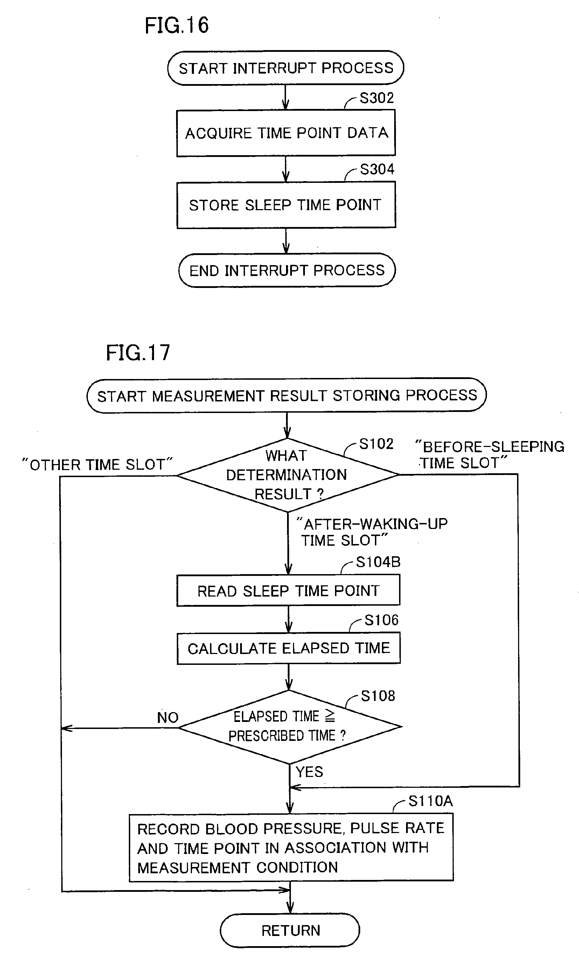

Fig. 16 is the flowchart showing the flow of an interrupt process started when sleep time switch 9 is manipulated.

Referring to Fig. 16, CPU 20 acquires time point data from timer 13 (S302). Based on the acquired time point data, it stores sleep time point data in a prescribed area in memory 12, for example (S304). Thus, the interrupt process is ended. It is noted that in S304 data of year, month, day, and time point is stored as the sleep time point data.

Fig. 17 is a flowchart showing a measurement result storing process in the second modification of the second embodiment. The processes similar to those in Fig. 13 used in the second embodiment have allotted the same step numbers, and description thereof is not repeated. The measurement result storing process shown in the following may be performed in S 18A in Fig. 4 of the first embodiment.

Referring to Fig. 17, in the second modification, the process of S104B is performed in place of the process of S 104A in Fig. 13. That is, in S 104B, time determining unit 212 of CPU 20 reads sleep time point data of the same evaluation day recorded in memory 12. Then in S106, the elapsed time from the sleep time point is calculated.

In the second modification of the second embodiment also, in S110A, while data of blood pressure value, pulse rate, and time point is stored in memory, only the blood pressure value and the pulse rate may be stored herein.

In the flowchart of Fig. 17, while the description has been given assuming that the sleep time point of the same evaluation day is stored, when the sleep time point of the same evaluation day is not stored, for example processes of S 106 and S108 may be skipped and the process of S110A may be performed. Alternatively, since whether the prescribed condition is satisfied cannot be determined if the sleep time point of the same evaluation day is not stored, the routine may be ended without performing the processes of S106-S110A.

Further, as a still another modification, the first modification of the second embodiment and the second modification of the second embodiment may be combined. In this case, in S108, when it is determined by time determining unit 212 that the elapsed time from the sleeping time is at least a prescribed time, the value of calculation flag F is set to 0 (S202), similarly to the first modification. In S108, if it is determined that the elapsed time from the sleeping time is less than a prescribed time, the value of calculation flag F is set to 1 similarly to the first modification (S202). When the process of S202 or S204 is ended, in place of the process of S110C, CPU 20 stores in memory 12 the blood pressure value, pulse rate and calculation flag value in association with the measurement conditions.

In the second embodiment and its first and second modifications, in storing the measurement results in memory 12, they are classified based on whether they are used for calculating the evaluation quantity. The measurement results similar to the first embodiment may be stored in record R and classified when calculating the risk value. That is, whether a prescribed condition is satisfied may be determined based on measurement time point data T stored in association with the measurement results in each area of memory 12. In this case also, a process similar to the interrupt process shown in Fig. 11 in the first embodiment may be performed. That is, in S54, CPU 20 determines whether there are at least three pieces of blood pressure data determined to satisfy a prescribed condition. Thus, a further reliable evaluation quantity can be calculated.

The risk value calculation method performed by the electronic blood pressure monitor of the present invention can be provided as a program. The program can be provided as a program product by recording in an optical medium such as a CD-ROM (Compact Disc-ROM) or a computer readable recording medium such as a memory card. Also, the program can be provided by downloading through a network.

The provided program product is installed in a program storing unit such as hard disk and executed. It is noted that the program product includes the program itself and the recording medium recording the program.

Although the present invention has been described and illustrated in detail, it is clearly understood that the same is by way of illustration and example only and is not to be taken by way of limitation, the spirit and scope of the present invention being limited only by the terms of the appended claims.

**Claims**

1. An electronic blood pressure monitor, comprising:

    a measuring unit (200) measuring a blood pressure of a subject;
    a data discriminating unit (211) discriminating whether measured blood pressure data is calculation data for calculating an evaluation quantity;
    a storing unit (12) storing said measured blood pressure data in association with at least one of a plurality of measurement conditions;
    a first retrieving unit (203) retrieving a plurality of pieces of blood pressure data measured in a prescribed period from the blood pressure data stored in said storing unit; and
    a first determining unit (205) determining whether at least a plurality of prescribed number of pieces of blood pressure data corresponding to each of said plurality of measurement conditions are present in said plurality of pieces of blood pressure data retrieved by said first retrieving unit, wherein
    said first determining unit performs said determination for the blood pressure data discriminated as said calculation data by said data discriminating unit, and said electronic blood pressure monitor further includes:

        a first average value calculating unit (207) calculating, when said first determining unit determines that at least the plurality of prescribed number of pieces of blood pressure data are present, a first average value of a blood pressure data group that includes said at least the plurality of prescribed number of pieces of blood pressure data, for each of the measurement conditions;

a first evaluation quantity calculating unit (208) calculating a first correlated evaluation quantity based on correlation of said first average values respectively calculated for said measurement conditions; and

a display unit (4) displaying a calculation result of said first evaluation quantity calculating unit.

2. The blood pressure monitor according to claim 1, wherein
said plurality of measurement conditions include an after-waking-up time slot and a before-sleeping time slot, and said blood pressure monitor further includes:

a time measuring unit measuring a time point;
a time point specifying unit specifying a sleep time point based on an instruction from the subject; and
a condition determining unit determining to which of said plurality of measurement conditions a measurement condition at the blood pressure measurement applies, based on time point data output from said time measuring unit, wherein
said data discriminating unit includes a time determining unit determining whether a prescribed time has elapsed from said sleep time point specified by said time point specifying unit, based on the time point data output from said time measuring unit, when said condition determining unit determines that said measurement condition is said after-waking-up time slot, and
said data discriminating unit discriminates said measured blood pressure data as said calculation data, when said time determining unit determines that said prescribed time has elapsed.

3. The blood pressure monitor according to claim 2, wherein
said prescribed period is a period that is an evaluation day including one said after-waking-up time slot and one said before-sleeping time slot.

4. The blood pressure monitor according to claim 1, wherein
said plurality of measurement conditions include an after-waking-up time slot and a before-sleeping time slot, and said blood pressure monitor further includes:

a time measuring unit measuring a time point; and
a condition determining unit determining to which of said plurality of measurement conditions a measurement condition at the blood pressure measurement applies, based on time point data output from said time measuring unit, wherein
said data discriminating unit includes a time determining unit determining whether a prescribed time has elapsed from a time point as measured in said before-sleeping time slot in said prescribed period, based on the time point data output from said time measuring unit, when said condition determining unit determines that said measurement condition is said after-waking-up time slot, and
said data discriminating unit discriminates said measured blood pressure data as said calculation data, when said time determining unit determines that said prescribed time has elapsed.

5. The blood pressure monitor according to claim 4, wherein
said prescribed period is a period that is an evaluation day including one said after-waking-up time slot and one said before-sleeping time slot.

6. The blood pressure monitor according to claim 1, wherein
the blood pressure data discriminated as said calculation data by said data discriminating unit is stored in said storing unit in association with said measurement conditions.

7. The blood pressure monitor according to claim 1, wherein
based on a discrimination result of said data discriminating unit, identification information indicating whether the blood pressure data is said calculation data and said measured blood pressure data are stored in said storing unit in association with said measurement conditions.

8. The blood pressure monitor according to claim 7, wherein
said first determining unit determines whether at least the plurality of prescribed number of pieces of blood pressure data, which correspond to each of said plurality of measurement conditions and in which said identification information indicates that the blood pressure data is said calculation data, are present, in said retrieved plurality of pieces of blood pressure data.

9. The blood pressure monitor according to claim 1, further comprising:

a second retrieving unit (204) retrieving a plurality of blood pressure data measured in a specific period that is longer than said prescribed period, from said blood pressure data stored in said storing unit;

a second determining unit (206) determining whether at least said plurality of prescribed number of pieces of blood pressure data corresponding to each of said plurality of measurement conditions for each said prescribed period are present in said plurality of pieces of blood pressure data retrieved by said second retrieving unit;

a second average value calculating unit (208) excluding blood pressure data measured in a period determined by said second determining unit that at least the plurality of prescribed number of pieces of blood pressure data are not present, to calculate a second average value for each of a plurality of blood pressure data groups, for each of the measurement conditions; and

a second evaluation quantity calculating unit (202) calculating a second correlated evaluation quantity based on correlation of said second average values for each of the measurement conditions.

10. The blood pressure monitor according to claim 9, wherein
said specific period is one of a week, a month, a season, and a year.

11. An electronic blood pressure monitor, comprising:

a measuring unit measuring a blood pressure of a subject;

a data discriminating unit discriminating whether measured blood pressure data is calculation data for calculating an evaluation quantity;

a storing unit storing said measured blood pressure data in association with at least one measurement condition;

a first retrieving unit retrieving a plurality of pieces of blood pressure data measured in a prescribed period from the blood pressure data stored in said storing unit; and

a first determining unit determining whether at least a plurality of prescribed number of pieces of blood pressure data corresponding to a prescribed measurement condition are present in said plurality of pieces of blood pressure data retrieved by said first retrieving unit, wherein

said first determining unit performs said determination for the blood pressure data discriminated as said calculation data by said data discriminating unit, and said electronic blood pressure monitor further includes:

a first average value calculating unit calculating, when said first determining unit determines that at least the plurality of prescribed number of pieces of blood pressure data are present, a first average value of a blood pressure data group including said at least the plurality of prescribed number of pieces of blood pressure data, for said prescribed measurement condition;

a first evaluation quantity calculating unit calculating a first evaluation quantity based on said first average value; and

a display unit displaying a calculation result of said first evaluation quantity calculating unit.

12. The blood pressure monitor according to claim 11, wherein
said prescribed measurement condition corresponds to an after-waking-up time slot, and
said blood pressure monitor further includes:

a time measuring unit measuring time point;

a time point specifying unit specifying a sleep time point based on an instruction from the subject; and

a condition determining unit determining whether a measurement condition at the blood pressure measurement applies to said at least one measurement condition, based on time point data output from said time measuring unit, wherein

said data discriminating unit includes a time determining unit determining whether a prescribed time has elapsed from said sleep time point specified by said time point specifying unit, based on the time point data output from said time measuring unit, when said condition determining unit determines that said measurement condition is said prescribed measurement condition, and

said data discriminating unit discriminates said measured blood pressure data as said calculation data, when said time determining unit determines that said prescribed time has elapsed.

13. The blood pressure monitor according to claim 12, wherein
said prescribed period is a period that is an evaluation day including one said after-waking-up time slot.

**14.** The blood pressure monitor according to claim 11, wherein
said prescribed measurement condition corresponds to an after-waking-up time slot, and
said blood pressure monitor further includes:

a time measuring unit measuring a time point; and
a condition determining unit determining whether a measurement condition at the blood pressure measurement applies to said at least one measurement condition, based on time point data output from said time measuring unit, wherein
said data discriminating unit includes a time determining unit determining whether a prescribed time has elapsed from a time point as measured in a before-sleeping time slot in said prescribed period, based on the time point data output from said time measuring unit, when said condition determining unit determines that said measurement condition is said prescribed measurement condition, and
said data discriminating unit discriminates said measured blood pressure data as said calculation data, when said time determining unit determines that said prescribed time has elapsed.

**15.** The blood pressure monitor according to claim 14, wherein
said prescribed period is a period that is an evaluation day including one said after-waking-up time slot.

**16.** The blood pressure monitor according to claim 11, wherein
the blood pressure data discriminated as said calculation data by said data discriminating unit is stored in said storing unit in association with said prescribed measurement condition.

**17.** The blood pressure monitor according to claim 11, wherein
based on a discrimination result of said data discriminating unit, identification information indicating whether the blood pressure data is said calculation data and said measured blood pressure data are stored in said storing unit in association with said prescribed measurement condition.

**18.** The blood pressure monitor according to claim 17, wherein
said first determining unit determines whether at least the plurality of prescribed number of pieces of blood pressure data, which correspond to said prescribed measurement condition and in which said identification information indicates that the blood pressure data is said calculation data, are present, in said retrieved plurality of pieces of blood pressure data.

**19.** The blood pressure monitor according to claim 11, further comprising:

a second retrieving unit retrieving a plurality of blood pressure data measured in a specific period that is longer than said prescribed period, from said blood pressure data stored in said storing unit;
a second determining unit determining whether at least said plurality of prescribed number of pieces of blood pressure data corresponding to said prescribed measurement condition for each said prescribed period are present in said plurality of pieces of blood pressure data retrieved by said second retrieving unit;
a second average value calculating unit excluding blood pressure data measured in a period determined by said second determining unit that at least the plurality of prescribed number of pieces of blood pressure data are not present, to calculate a second average value for each of a plurality of blood pressure data groups, for said prescribed measurement condition; and
a second evaluation quantity calculating unit calculating a second correlated evaluation quantity based on correlation of said second average values for said prescribed measurement condition.

**20.** The blood pressure monitor according to claim 19, wherein
said specific period is one of a week, a month, a season, and a year.

**21.** A method of processing blood pressure measurement data, comprising:

a measuring step of measuring a blood pressure of a subject;
a data discriminating step of discriminating whether measured blood pressure data is calculation data for calculating an evaluation quantity;
a storing step of storing in a memory said measured blood pressure data in association with at least one of a plurality of measurement conditions;
a first retrieving step of retrieving a plurality of pieces of blood pressure data measured in a prescribed period from the blood pressure data stored in said memory; and

a first determining step of determining whether at least a plurality of prescribed number of pieces of blood pressure data corresponding to each of said plurality of measurement conditions are present in said plurality of pieces of blood pressure data retrieved by said first retrieving step, wherein

in said first determining step, said determination is performed for the blood pressure data discriminated as said calculation data by said data discriminating step, and said method further includes:

a first average value calculating step of calculating, when it is determined by said first determining step that at least the plurality of prescribed number of pieces of blood pressure data are present, a first average value of a blood pressure data group including said at least the plurality of prescribed number of pieces of blood pressure data, for each of the measurement conditions;

a first evaluation quantity calculating step of calculating a first correlated evaluation quantity based on correlation of said first average values respectively calculated for said measurement conditions; and

a display step of displaying a calculation result of said first evaluation quantity calculating step.

22. A method of processing blood pressure measurement data, comprising:

a measuring step of measuring a blood pressure of a subject;

a data discriminating step of discriminating whether measured blood pressure data is calculation data for calculating an evaluation quantity;

a storing step of storing in a memory said measured blood pressure data in association with at least one measurement condition;

a first retrieving step of retrieving a plurality of pieces of data measured in a prescribed period from the blood pressure data stored in said memory; and

a first determining step of determining whether at least a plurality of prescribed number of pieces of blood pressure data corresponding to a prescribed measurement condition are present in said plurality of pieces of blood pressure data retrieved by said first retrieving step, wherein

in said first determining step, said determination is performed for the blood pressure data discriminated as said calculation data by said data discriminating step, and said method further includes:

a first average value calculating step of calculating, when it is determined by said first determining step that at least the plurality of prescribed number of pieces of blood pressure data are present, a first average value of a blood pressure data group including said at least the plurality of prescribed number of pieces of blood pressure data, for said prescribed measurement condition;

a first evaluation quantity calculating step of calculating a first evaluation quantity based on said first average value; and

a display step of displaying a calculation result of said first evaluation quantity calculating step.

FIG.1

**FIG.2**

FIG.3B

12

| | R1 |
|---|---|
| T1, SBP1, DBP1, PLS1, C1 | |
| T2, SBP2, DBP2, PLS2, C1 | R2 |
| T3, SBP3, DBP3, PLS3, C1 | R3 |
| T4, SBP4, DBP4, PLS4, C2 | R4 |
| ... | ... |
| Ti, SBPi, DBPi, PLSi, C1 | Ri |
| ... | ... |
| Tn, SBPn, DBPn, PLSn, C2 | Rn |

FIG.3A

12

T, SBP, DBP, PLS — R

BEFORE-SLEEPING
MEASUREMENT AREA — 26

AFTER-WAKING-UP
MEASUREMENT AREA — 27

## FIG.4

START (POWER ON)

S1 — INITIALIZATION

S2 — MEASUREMENT TIME INFORMATION STORED ? — NO

YES

S3 — CHANGE ? — NO

YES

S4 — ACCEPT INPUT OF MEASUREMENT CONDITION SPECIFYING INFORMATION

S5 — RECORD MEASUREMENT TIME INFORMATION

S6 — MEASUREMENT SWITCH MANIPULATED ? — NO

YES

S8 — RAISE CUFF PRESSURE

S10 — REDUCE CUFF PRESSURE

S12 — ·CALCULATE BLOOD PRESSURE ·CALCULATE PULSE RATE

S14 — ·DISPLAY BLOOD PRESSURE ·DISPLAY PULSE RATE

S16 — DETERMINE CURRENT MEASUREMENT CONDITION

S18A — MEASUREMENT RESULT STORING PROCESS

S19 — RETRIEVE MESUREMENT RESULT OF THE SAME EVALUATION DAY

S20 — AT LEAST THREE MEASUREMENT RESULTS CORRESPONDING TO EACH MEASUREMENT CONDITION PRESENT ? — YES / NO

S22 — CALCULATE AVERAGE VALUE FOR EACH MEASUREMENT CONDITION

S24 — CALCULATE RISK VALUE

S26 — DISPLAY RISK VALUE

END

FIG.5

START MEASUREMENT RESULT STORING PROCESS

"OTHER TIME SLOT"

WHAT DETERMINATION RESULT ?  S32

"BEFORE-SLEEPING TIME SLOT"
"AFTER-WAKING-UP TIME SLOT"

S34

RECORD BLOOD PRESSURE, PULSE RATE AND TIME POINT IN ASSOCIATION WITH MEASUREMENT CONDITION

RETURN

## FIG.6A

```
┌──────────────────────────── 4
│                                    │
│   MEASUREMENT CONDITION INPUT SCREEN   │
│                                    │
│   WAKE UP TIME        7  :  00     │
│                                    │
│                                    │
│   SLEEPING TIME      21  :  00     │
│                                    │
│                                    │
│                                    │
└────────────────────────────────────┘
```

## FIG.6B

```
┌──────────────────────────── 4
│                                    │
│   MEASUREMENT CONDITION INPUT SCREEN   │
│                                    │
│   AFTER-WAKING-UP TIME SLOT        │
│                                    │
│        5  :  30   ~    7  :  00    │
│                                    │
│   BEFORE-SLEEPING TIME SLOT        │
│                                    │
│       21  :  00   ~   22  :  00    │
│                                    │
└────────────────────────────────────┘
```

## FIG.6C

```
┌──────────────────────────── 4
│                                    │
│   MEASUREMENT CONDITION INPUT SCREEN   │
│                                    │
│  ○ WAKE UP TIME       7  :  00     │
│       TIME DURATION   1  :  30     │
│  ○ SLEEPING TIME     21  :  00     │
│       TIME DURATION   1  :  00     │
│                                    │
│                                    │
└────────────────────────────────────┘
```

27

## FIG.7A

| MEASUREMENT CONDITION | TIME | TIME DURATION |
|---|---|---|
| AFTER WAKING UP | 7:00 | 1:30 |
| BEFORE SLEEPING | 21:00 | 1:00 |

## FIG.7B

| MEASUREMENT CONDITION | TIME SLOT |
|---|---|
| AFTER WAKING UP | 5:30～7:00 |
| BEFORE SLEEPING | 21:00～22:00 |

## FIG.8

ME DIFFERENCE

REFERENCE VALUE:20mmHg

ME AVERAGE

REFERENCE VALUE:135mmHg

**FIG.9**

**FIG.10**

## FIG.11

```
        ┌─────────────────────────────────┐
        │    START INTERRUPT PROCESS       │
        └─────────────────────────────────┘
                        │
                        ▼            ⌐S52
        ┌─────────────────────────────────┐
        │ READ MEASUREMENT RESULTS         │
        │ FOR ONE WEEK                     │
        └─────────────────────────────────┘
                        │
                        ▼            ⌐S54
        ┌─────────────────────────────────┐
        │ DETERMINE WHETHER AT LEAST       │
        │ A PRESCRIBED NUMBER OF           │
        │ MEASUREMENT RESULTS              │
        │ CORREPSONDING TO EACH            │
        │ MEASUREMENT CONDITION ARE        │
        │ PRESENT ON EVALUATION DAY        │
        │ BASIS                            │
        └─────────────────────────────────┘
                        │
                        ▼            ⌐S56
        ┌─────────────────────────────────┐
        │ EXCLUDE DAY WITH LESS THAN       │
        │ THE PRESCRIBED NUMBER            │
        └─────────────────────────────────┘
                        │
                        ▼            ⌐S58
        ┌─────────────────────────────────┐
        │ CALCULATE AVERAGE VALUE          │
        │ FOR EACH EVALUATION DAY          │
        └─────────────────────────────────┘
                        │
                        ▼            ⌐S60
        ┌─────────────────────────────────┐
        │    CALCULATE RISK VALUE          │
        └─────────────────────────────────┘
                        │
                        ▼            ⌐S62
        ┌─────────────────────────────────┐
        │      DISPLAY RISK VALUE          │
        └─────────────────────────────────┘
                        │
                        ▼
        ┌─────────────────────────────────┐
        │     END INTERRUPT PROCESS        │
        └─────────────────────────────────┘
```

# FIG.12

START (POWER ON)

↓ S1

INITIALIZATION

↓ S4

ACCEPT INPUT OF MEASUREMENT CONDITION SPECIFYING INFORMATION

↓

S6 MEASUREMENT SWITCH MANIPULATED ?

NO → (loop back)

YES ↓ S8

RAISE CUFF PRESSURE

↓ S10

REDUCE CUFF PRESSURE

→

S12
· CALCULATE BLOOD PRESSURE
· CALCULATE PULSE RATE

↓ S14
· DISPLAY BLOOD PRESSURE
· DISPLAY PULSE RATE

↓ S16

DETERMINE CURRENT MEASUREMENT CONDITION

↓ S18B

MEASUREMENT RESULT STORING PROCESS

↓ S19

RETRIEVE MESUREMENT RESULT OF THE SAME EVALUATION DAY

↓ S20

AT LEAST THREE MEASUREMENT RESULTS CORRESPONDING TO EACH MEASUREMENT CONDITION PRESENT ?

NO →

YES ↓ S22

CALCULATE AVERAGE VALUE FOR EACH MEASUREMENT CONDITION

↓ S24

CALCULATE RISK VALUE

↓ S26

DISPLAY RISK VALUE

↓

END

## FIG.13

START MEASUREMENT RESULT STORING PROCESS

"OTHER TIME SLOT" ← S102 WHAT DETERMINATION RESULT ? → "BEFORE-SLEEPING TIME SLOT"

"AFTER-WAKING-UP TIME SLOT"

S104A
RETRIEVE LATEST MEASUREMENT TIME POINT DATA IN BEFORE-SLEEPING TIME SLOT IN THE SAME EVALUATION DAY

S106
CALCULATE ELAPSED TIME

S108
NO ← ELAPSED TIME ≧ PRESCRIBED TIME ?

YES

S110A
RECORD BLOOD PRESSURE, PULSE RATE AND TIME POINT IN ASSOCIATION WITH MEASUREMENT CONDITION

RETURN

FIG.14B

122

RR1 — T1、F、SBP1、DBP1、PLS1、C1
RR2 — T2、F、SBP2、DBP2、PLS2、C1
RR3 — T3、F、SBP3、DBP3、PLS3、C1
RR4 — T4、F、SBP4、DBP4、PLS4、C2
···
RRi — Ti、F、SBPi、DBPi、PLSi、C1
···
RRn — Tn、F、SBPn、DBPn、PLSn、C2

FIG.14A

122

RR — T、F、SBP、DBP、PLS

26 BEFORE-SLEEPING MEASUREMENT AREA

27 AFTER-WAKING-UP MEASUREMENT AREA

# FIG.15

START MEASUREMENT RESULT STORING PROCESS

"OTHER TIME SLOT" ← WHAT DETERMINATION RESULT ? → S102 "BEFORE-SLEEPING TIME SLOT"

"AFTER-WAKING-UP TIME SLOT"

S104A
RETRIEVE LATEST MEASUREMENT TIME POINT DATA IN BEFORE-SLEEPING TIME SLOT IN THE SAME EVALUATION DAY

S106
CALCULATE ELAPSED TIME

S108
ELAPSED TIME ≧ PRESCRIBED TIME ? —— YES

NO

S202
CALCULATION FLAG VALUE ← 0

S204
CALCULATION FLAG VALUE ← 1

S110B
RECORD BLOOD PRESSURE, PULSE RATE, CALCULATION FLAG VALUE, AND TIME POINT IN ASSOCIATION WITH MEASUREMENT CONDITION

RETURN

34

## FIG.16

START INTERRUPT PROCESS

⌐S302

ACQUIRE TIME POINT DATA

⌐S304

STORE SLEEP TIME POINT

END INTERRUPT PROCESS

## FIG.17

START MEASUREMENT RESULT STORING PROCESS

⌐S102

"OTHER TIME SLOT"          WHAT DETERMINATION RESULT ?          "BEFORE-SLEEPING TIME SLOT"

"AFTER-WAKING-UP TIME SLOT"

⌐S104B

READ SLEEP TIME POINT

⌐S106

CALCULATE ELAPSED TIME

⌐S108

NO          ELAPSED TIME ≧ PRESCRIBED TIME ?

YES

⌐S110A

RECORD BLOOD PRESSURE, PULSE RATE AND TIME POINT IN ASSOCIATION WITH MEASUREMENT CONDITION

RETURN

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- JP 4221528 A

- JP 3033849 B

### Non-patent literature cited in the description

- **KAZUOMI KARIO.** Risk of Morning Hypertension and Cerebrovascular Disease. *Journal of Blood Pressure,* 01 November 2002, vol. 9 (11), 94-97
- Guidelines for the Management of Hypertension. Japanese Society of Hypertension, 20 December 2004, 7-8

- Number of Measurements. Hypertension. American Heart Association, January 2005, vol. 45, 151-152